(19) 

Europäisches Patentamt
European Patent Office
Office européen des brevets

(11) **EP 4 703 380 A1**

(12) **EUROPEAN PATENT APPLICATION**
published in accordance with Art. 153(4) EPC

(43) Date of publication:
04.03.2026 Bulletin 2026/10

(21) Application number: 23935528.2

(22) Date of filing: 18.12.2023

(51) International Patent Classification (IPC):
*C07K 16/28* (2006.01)    *C07K 16/30* (2006.01)
*A61K 39/00* (2006.01)    *A61P 35/00* (2006.01)
*G01N 33/574* (2006.01)   *A61K 47/68* (2017.01)

(52) Cooperative Patent Classification (CPC):
A61K 39/00; A61K 47/68; A61P 35/00;
C07K 16/28; C07K 16/30; G01N 33/575

(86) International application number:
PCT/KR2023/020864

(87) International publication number:
WO 2024/225556 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.04.2023 KR 20230056672
22.05.2023 KR 20230065836

(71) Applicant: NIO BIOPHARMACEUTICALS. INC.
Seoul 02455 (KR)

(72) Inventors:
• KIM, Kristine M.
Chuncheon-si, Gangwon-do 24278 (KR)
• LEE, Jung Hyeon
Uijeongbu-si, Gyeonggi-do 11631 (KR)
• KIM, Min Ji
Icheon-si, Gyeonggi-do 17353 (KR)

(74) Representative: Meissner Bolte Partnerschaft
mbB
Patentanwälte Rechtsanwälte
Postfach 86 06 24
81633 München (DE)

Remarks:
The complete document including Reference
Table(s) and the Sequence Listing(s) can be
downloaded from the EPO website

(54) **INTEGRIN-TARGETING ANTIBODIES AND USE THEREOF**

(57) The present invention relates to a cancer cell-specific antibody and its use. According to one aspect, an antibody specific to a membrane protein of a cancer stem cell or a cancer cell exhibits strong affinity for ITGA3 or VLA3 and excellent cell internalization, and thus can be usefully used in research on diagnosis, treatment, or development of therapeutic agents for various cancers in the form of unconjugated antibodies, antibody-drug conjugates (ADCs), and other antibody complexes based on the antibody.

Fig. 1

## Description

### Technical Field

The present invention relates to antibodies specific to cancer cells and uses thereof.

### Background Art

Cancer is a leading cause of death, and the number of patients is expected to increase with the aging population. However, the treatment needs are still not sufficiently met. Conventional chemotherapeutic agents have been problematic due to side effects caused by their cytotoxicity not only to tumor cells but also to normal cells. Furthermore, the drug's full effect cannot be achieved due to the inability to administer sufficient doses. Consequently, recent efforts have focused on developing more selective molecular-targeting drugs or antibody therapeutics that target molecules exhibiting characteristic mutations or overexpression in cancer cells, or specific molecules involved in cell carcinogenesis.

Antibodies have high blood stability and bind specifically to target antigens, so they are expected to reduce side effects, and many antibody drugs are being developed for molecules highly expressed on the surface of cancer cells. One such technology utilizing the antigen-specific binding ability of antibodies is the antibody-drug conjugate (ADC). ADC is an antibody conjugated to a cytotoxic drug that can bind to an antigen expressed on the surface of cancer cells and internalize the antigen into the cell through that binding. ADC is expected to effectively deliver drugs to cancer cells, accumulate the drugs within them, and thereby kill the cancer cells.

Characteristics of a target antigen suitable for ADC as an anti-tumor drug include: high expression specifically on the surface of cancer cells, low or no expression in normal cells, the ability to be internalized into cells, and the antigen not being secreted from the cell surface. Furthermore, an important characteristic of antibodies suitable for ADCs is their high internalization ability, in addition to their specific binding to the target antigen. The internalization ability of an antibody depends on the properties of both the target antigen and the antibody, and it is difficult to estimate an antigen-binding site suitable for internalization from the molecular structure of the target, or to easily infer an antibody with a high internalization ability from the binding strength or physical properties of the antibody. Therefore, obtaining an antibody with a high internalization ability against a target antigen has become a critical challenge in developing highly effective ADCs.

## Detailed Description of the Invention

### Technical Challenges

Accordingly, the inventors have developed a substance applicable to the development of antibody-based pharmaceuticals, such as antibody-drug conjugates, by identifying antibodies that bind to and internalize into target antigens reflecting phenotypic structural characteristics, thereby addressing the issues in current cancer drug development and treatment methods.

One aspect of the present invention is to provide an antibody or functional fragment thereof specific to a membrane protein of cancer stem cells or cancer cells, comprising: (a) a VH CDR1 containing an amino acid sequence of any one of SEQ ID Nos: 1 to 4; (b) a VH CDR2 containing an amino acid sequence of any one of SEQ ID Nos: 5 to 8; (c) a VH CDR3 containing an amino acid sequence of any one of SEQ ID Nos: 9 to 12; (d) a VL CDR1 containing an amino acid sequence of any one of SEQ ID Nos: 13 to 16; (e) a VL CDR2 containing an amino acid sequence of any one of SEQ ID Nos: 17 to 20; and (f) a VL CDR3 containing an amino acid sequence of any one of SEQ ID Nos: 21 to 24.

Another aspect is to provide a pharmaceutical composition for treating or preventing cancer, comprising the antibody or functional fragment thereof.

Another aspect is to provide a chimeric antigen receptor (CAR) protein comprising the antibody or functional fragment thereof.

Another aspect is to provide immune cells comprising the chimeric antigen receptor protein.

Another aspect is to provide a polynucleotide encoding the antibody or functional fragment thereof.

Another aspect is to provide a recombinant vector comprising the above polynucleotide.

Another aspect is to provide a recombinant cell comprising the above vector.

Another aspect is to provide a composition for diagnosing cancer comprising the antibody or functional fragment thereof.

Another aspect is to provide a cancer diagnostic kit comprising the composition.

Another aspect is to provide a reagent composition comprising the antibody or the polynucleotide.

Another aspect is to provide a method for treating or preventing cancer, comprising a step of administering an effective amount of the antibody or functional fragment thereof to a subject in need thereof.

**Means of Solving Problems**

One aspect of the present invention is to provide an antibody or functional fragment thereof specific to a membrane protein of a cancer stem cell or a cancer cell, comprising: (a) a VH CDR1 containing an amino acid sequence of any one of SEQ ID NOs: 1 to 4; (b) a VH CDR2 containing an amino acid sequence of any one of SEQ ID NOs: 5 to 8; (c) a VH CDR3 containing an amino acid sequence of any one of SEQ ID NOs: 9 to 12; (d) a VL CDR1 containing an amino acid sequence of any one of SEQ ID NOs: 13 to 16; (e) a VL CDR2 containing an amino acid sequence of any one of SEQ ID NOs: 17 to 20; and (f) a VL CDR3 containing an amino acid sequence of any one of SEQ ID NOs: 21 to 24.

In one specific embodiment, the antibody or functional fragment thereof may comprise: (a) a VH CDR1 of SEQ ID NO: 1, a VH CDR2 of SEQ ID NO: 5, a VH CDR3 of SEQ ID NO: 9, a VL CDR1 of SEQ ID NO: 13, a VL CDR2 of SEQ ID NO: 17, and a VL CDR3 of SEQ ID NO: 21; (b) a VH CDR1 of SEQ ID NO: 2, a VH CDR2 of SEQ ID NO: 6, a VH CDR3 of SEQ ID NO: 10, a VL CDR1 of SEQ ID NO: 14, a VL CDR2 of SEQ ID NO: 18, and a VL CDR3 of SEQ ID NO: 22; (c) a VH CDR1 of SEQ ID NO: 3, a VH CDR2 of SEQ ID NO: 7, a VH CDR3 of SEQ ID NO: 11, a VL CDR1 of SEQ ID NO: 15, a VL CDR2 of SEQ ID NO: 19, and a VL CDR3 of SEQ ID NO: 23; or (d) a VH CDR1 of SEQ ID NO: 4, a VH CDR2 of SEQ ID NO: 8, a VH CDR3 of SEQ ID NO: 12, a VL CDR1 of SEQ ID NO: 16, a VL CDR2 of SEQ ID NO: 20 and a VL CDR3 of SEQ ID NO: 24.

In one specific embodiment, the antibody or functional fragment thereof may comprise: (a) a heavy chain variable region containing an amino acid sequence of any one of SEQ ID NOs: 25 to 28 or a heavy chain variable region containing a sequence having at least 90% homology to an amino acid sequence of any one of SEQ ID NOs: 25 to 28; and (b) a light chain variable region containing an amino acid sequence of any one of SEQ ID NOs: 29 to 32 or a light chain variable region containing a sequence having at least 90% homology to an amino acid sequence of any one of SEQ ID NOs: 29 to 32.

In one specific embodiment, the antibody or functional fragment thereof may comprise: (a) a heavy chain variable region containing the amino acid sequence of SEQ ID NO: 25 or a heavy chain variable region containing a sequence having at least 90% homology to the amino acid sequence of SEQ ID NO: 25; and a light chain variable region containing the amino acid sequence of SEQ ID NO: 29 or a light chain variable region containing a sequence having at least 90% homology to the amino acid sequence of SEQ ID NO: 29; (b) a heavy chain variable region containing the amino acid sequence of SEQ ID NO: 26 or a heavy chain variable region containing a sequence having at least 90% homology to the amino acid sequence of SEQ ID NO: 26; and a light chain variable region containing the amino acid sequence of SEQ ID NO: 30 or a light chain variable region containing a sequence having at least 90% homology to the amino acid sequence of SEQ ID NO: 30; (c) a heavy chain variable region containing the amino acid sequence of SEQ ID NO: 27 or a heavy chain variable region containing a sequence having at least 90% homology to the amino acid sequence of SEQ ID NO: 27; and a light chain variable region containing the amino acid sequence of SEQ ID NO: 31 or a light chain variable region containing a sequence having at least 90% homology to the amino acid sequence of SEQ ID NO: 31; or (d) a heavy chain variable region containing the amino acid sequence of SEQ ID NO: 28 or a heavy chain variable region containing a sequence having at least 90% homology to the amino acid sequence of SEQ ID NO: 28; and a light chain variable region containing the amino acid sequence of SEQ ID NO: 32 or a light chain variable region containing a sequence having at least 90% homology to the amino acid sequence of SEQ ID NO: 32.

In one specific embodiment, the CDRs of each variable region of the light chain and each variable region of the heavy chain can be freely combined.

In one specific embodiment, the membrane protein of the cancer stem cell or cancer cell may be ITGA3 (Integrin Subunit Alpha 3) or VLA3 (Integrin alpha 3 beta 1).

Integrins are heterodimeric cell surface receptors that bind to ECM (extracellular matrix) proteins and are involved in cell adhesion, proliferation, and migration. Integrins can also recruit various proteins and influence signaling pathways, thereby regulating gene expression and cell survival.

Integrin proteins consist of 18 types of alpha ($\alpha$) subunits and 8 types of beta ($\beta$) subunits. A total of 24 types of integrins exist, consisting of various heterodimer combinations of $\alpha$ and $\beta$ subunits. Among these combinations, cancer-specific integrin complexes exist.

The nucleotide and protein sequences of ITGA3 are known for many species. For example, the human sequence is NCBI accession number NG_029107.2 and Uniprot accession number P23006.

The ITGA3 and VLA3 mentioned above include variants, isoforms, homologs, orthologs, and paralogs. For example, antibodies specific to human ITGA3 or VLA3 proteins may, in certain cases, cross-react with ITGA3 or VLA3 proteins from non-human species.

In one specific embodiment, the antibody or functional fragment thereof may be selected from the group consisting of IgG, Fab, Fab', F(ab')2, xFab, scFab, dsFv, Fv, scFv, scFv-Fc, scFab-Fc, diabody (scFv2, diabody), minibody, scAb, dAb (domain antibody, VH or VL), Fc-scFv, scFv-Fc-scFv, IgG-scFv, scFv-IgG and combinations thereof. Specifically, the antibody or functional fragment thereof may be a diabody, a dAb, a scFv or a scFv-Fc.

As used herein, a "Fab fragment" consists of one light chain and one heavy chain, each containing only the variable region and CH1. The heavy chain of a Fab molecule cannot form a disulfide bond with another heavy chain molecule. A scFab is formed by connecting two Fab molecules via a flexible linker.

In this specification, a "Fab' fragment" includes a region between the CH1 and CH2 domains of a heavy chain, in addition to the Fab fragment itself, and can form a disulfide bond between the two heavy chains of two Fab' fragment molecules to generate F(ab')2 molecules.

As used herein, a "F(ab')2 fragment" comprises two light chains and two heavy chains, each containing the variable regions, CH1, and a portion of the constant region between the CH1 and CH2 domains as mentioned above, and thus comprises two intrachain disulfide bonds. Accordingly, the F(ab')2 fragment consists of two Fab' fragments, which are joined to each other by a disulfide bond therebetween.

As used herein, the term "cross-Fab fragment" or "xFab fragment" or "crossover Fab fragment" refers to a Fab fragment in which the variable regions or constant regions of the heavy and light chains are exchanged. Two different chain compositions of crossover Fab molecules are possible. The variable regions of the Fab heavy and light chains can be exchanged. That is, a crossover Fab molecule comprises a peptide chain consisting of a light chain variable region (VL) and a heavy chain constant region (CH1), and a peptide chain consisting of a heavy chain variable region (VH) and a light chain constant region (CL). This crossover Fab molecule is also referred to as CrossFab(VLVH). On the other hand, when the constant regions of the Fab heavy and light chains are exchanged, the crossover Fab molecule comprises a peptide chain consisting of a heavy chain variable region (VH) and a light chain constant region (CL), and a peptide chain consisting of a light chain variable region (VL) and a heavy chain constant region (CH1). This crossover Fab molecule is also referred to as CrossFab(CLCH1).

As used herein, a "single chain Fab fragment" or "scFab" is a polypeptide comprising an antibody heavy chain variable domain (VH), an antibody constant domain 1 (CH1), an antibody light chain variable domain (VL), an antibody light chain constant domain (CL), and a linker. Wherein the antibody domains and the linker have one of the following sequences from N-terminal to C-terminal: a) VH-CH1-linker-VL-CL, b) VL-CL-linker-VH-CH1, c) VH -CL-linker-VL-CH1, or d) VL-CH1-linker-VH-CL; wherein the linker is a polypeptide of at least 30 amino acids, preferably 32 to 50 amino acids. The single chain Fab fragment is stabilized by a natural disulfide bond between the CL domain and the CH1 domain. Additionally, these single-chain Fab molecules can be further stabilized by the creation of interchain disulfide bonds through insertion of cysteine residues (e.g., at position 44 of the variable heavy chain and position 100 of the variable light chain according to Kabat numbering).

As used herein, the term "Fv region" refers to an antibody fragment comprising each variable region of the heavy and light chains, but excluding the constant region. A sdFV is a fragment in which the heavy and light chains are linked by a disulfide bond. A scFv is a fragment in which the single-stranded variable regions (scFv) of the heavy and light chains are linked via a flexible linker. A scFv-Fc, where Fc is linked to scFv, can be referred to as a maxibody. The minibody is a fragment in which CH3 is linked to scFv. The diabody comprises two molecules of scFv.

In one specific embodiment, the scFv may have a heavy chain variable region and a light chain variable region linked via a linker.

In one embodiment, the scFv comprised in the antibody can comprise a heavy chain variable region and a light chain variable region in any order. For example, the scFv comprised in the antibody can comprise, from N-terminus to C-terminus, a heavy chain variable region and a light chain variable region, optionally with a peptide linker therebetween, or alternatively, the antibody can comprise, from N-terminus to C-terminus, a light chain variable region and a heavy chain variable region, optionally with a peptide linker therebetween.

The term "peptide linker" as used herein may include any amino acid of 1 to 100, 2 to 50, or 10 to 25, and any kind of amino acid may be included without limitation. The peptide linker may include, for example, Gly, Asn, and/or Ser residues, and may also include neutral amino acids such as Thr and/or Ala. Amino acid sequences suitable for the peptide linker may be known in the relevant art. Meanwhile, the length of the peptide linker may be determined in various ways within a range that does not affect the function of the antibody. For example, the peptide linker may be formed by including a total of about 1 to about 100, about 2 to about 50, or about 5 to about 25 residues selected from the group consisting of Gly, Asn, Ser, Thr, and Ala. In one embodiment, the peptide linker can be (G4S)n, (SG4)n or G4(SG4)n, where "n" is generally a number between 1 and 10, and typically between 2 and 4. The peptide linker may be, for example, GGGGS (SEQ ID NO: 33), GGGGSGGGGS (SEQ ID NO: 34), SGGGGSGGGG (SEQ ID NO: 35), GGGGSGGGGSGGGG (SEQ ID NO: 36), GSPGSSSSGS (SEQ ID NO: 37), GGGGSGGGGSGGGGS (SEQ ID NO: 38), GSGSGSGS (SEQ ID NO: 39), GSGSGNGS (SEQ ID NO: 40), GGSGSGSG (SEQ ID NO: 41), GGSGSG (SEQ ID NO: 42), GGSG (SEQ ID NO: 43), GGSGNGSG (SEQ ID NO: 44), GGNGSGSG (SEQ ID NO: 45), or GGNGSG (SEQ ID NO: 46).

The above linker may include a mutation. The mutated linker may further include Cysteine (Cys, C) in the linker. The linker may include the amino acid sequence of GGGGSGGGSCGGGGS (SEQ ID NO: 47).

As used herein, a "short-chain antibody (scAb)" refers to a single polypeptide chain comprising one variable region of either the heavy or light chain constant region, wherein said variable region is the heavy and light chain variable regions connected by a flexible linker. The short-chain antibody may be referred to in U.S. Patent No. 5,260,203, which is incorporated herein by reference.

As used herein, a "domain antibody (dAb)" is an immunologically functional immunoglobulin fragment comprising only the variable region of a heavy chain or the variable region of a light chain.

In this specification, "full-length IgG" is defined as comprising essentially complete IgG, but does not necessarily possess all the features of a complete IgG. A Full-length IgG contains two heavy chains and two light chains. Each chain contains constant (C) and variable (V) regions, which can be broken down into domains designated CH1, CH2, CH3, VH, and CL, VL.

In one specific embodiment, the Fc may comprise an amino acid sequence derived from human, mouse, chicken, monkey or camelid.

In one specific embodiment, the antibody or functional fragment thereof may be a chimeric antibody, a humanized antibody, or a fully human antibody.

The term "chimeric antibody" refers to a molecule in which different portions of the antibody are derived from different animal species, such as an antibody having a variable region derived from a mouse monoclonal antibody and a human immunoglobulin constant region. Methods for producing chimeric antibodies are known in the art.

The term "humanized antibody" refers to an antibody molecule derived from a non-human species antibody that binds to a desired antigen, having one or more complementary determining regions (CDRs) from a non-human species and a framework region from a human immunoglobulin molecule. Often, framework residues in the human framework region will be substituted with corresponding residues from the CDR donor antibody to alter, and preferably improve, antigen binding. Such framework substitutions are identified by methods well known in the art, such as by modeling the interactions of CDR and framework residues to identify framework residues important for antigen binding and by sequence comparison to identify unusual framework residues at specific positions (see, e.g., Queen et al., U.S. Pat. No. 5,585,089, which is incorporated herein by reference in its entirety).

The term "fully human antibody" is particularly preferred for therapeutic treatment of human patients. Human antibodies can be produced by various methods known in the art, including phage display methods using antibody libraries derived from human immunoglobulin sequences.

In one specific embodiment, the antibody or functional fragment thereof may be a monoclonal antibody.

In one specific embodiment, the antibody or functional fragment thereof may be of the type IgG1, IgG2, IgG3, IgG4 or a combination thereof.

One aspect of the present invention provides an immunoconjugate comprising an anti-ITGA3 or anti-VLA3 antibody as described herein conjugated (chemically linked) to one or more therapeutic agents, such as a cytotoxic agent, a chemotherapeutic agent, a drug, a growth inhibitory agent, a toxin (e.g., a protein toxin, an enzymatically active toxin of bacterial, fungal, plant or animal origin, or a fragment thereof), or a radioactive isotope.

In one specific embodiment, the immunoconjugate is an antibody-drug conjugate (ADC) in which an antibody is conjugated to one or more of the therapeutic agents mentioned above. The antibody-drug conjugate may be one in which two molecules are directly linked, or one indirectly linked to two molecules by any means such as a linker. The linker may be a non-cleavable linker or a cleavable linker. It is generally known that ADCs are ideally designed so that the drug can be cleaved inside the targeted cell after being indirectly linked by means such as a linker. The linker may be a peptide linker that can be cleaved by a cleavage agent present in the intracellular environment, for example, in a lysosome or endosome, and may be cleaved by, for example, an intracellular peptidase or protease enzyme, for example, a lysosomal or endosomal protease. The peptide linker generally has a length of at least two amino acids. For example, the cleavable linker may be pH sensitive, and may be susceptible to hydrolysis at a specific pH value. In general, a pH-sensitive linker indicates that it can be hydrolyzed under acidic conditions. For example, an acid-labile linker that can be hydrolyzed in lysosomes can be a hydrazone, a semicarbazone, a thiosemicarbazone, a cis-aconitic amide, an orthoester, an acetal, a ketal, etc. As another example, the linker can also be cleaved under reducing conditions, for example, a disulfide linker may fall into this category. Various disulfide bonds can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), and SMPT (N-succinimidyl-oxycarbonyl-alpha-methyl-alpha-(2-pyridyl-dithio)toluene).

The above therapeutic agent may be at least one selected from the group consisting of: (a) erlotinib, bortezomib, fulvestrant, sutent, letrozole, imatinib mesylate, PTK787/ZK 222584, oxaliplatin, 5-fluorouracil, leucovorin, rapamycin, lapatinib, lonafarnib, sorafenib, gefitinib, AG1478, AG1571, thiotepa, cyclophosphamide, busulfan, improsulfan, piposulfan, benzodopa, Carboquone, meturedopa, uredopa, ethylenimine, altretamine, triethylenemelamine, triethylenephosphoramide, triethylenethiophosphoramide, trimethylolomelamine, bullatacin, bullatacinone, camptothecin, topotecan, bryostatin, calystatin, CC-1065, adozelesin, carzelesin, bizelesin, cryptophycin 1, cryptophycin 8, dolastatin, Duocarmycin, KW-2189, CB1-TM1, eleutherobin, pancratistatin, sarcodictyin, spongistatin, chlorambucil, chlornaphazine, chlorophosphamide, estramustine, ifosfamide, mechlorethamine, melphalan, novembichin, phenesterine, prednimustine, trofosfamide, uracil mustard, carmustine, chlorozotocin, fotemustine, Lomustine, nimustine, ranimnustine, calicheamicin, calicheamicin gamma 1, calicheamicin omega 1, dynemicin, dynemicin A, clodronate, esperamicin, neocarzinostatin chromophore, aclacinomysins, actinomycin, antrmycin, azaserine, bleomycins, cactinomycin, carabicin, carninomycin, Carzinophilin, chromomycins, dactinomycin, daunorubicin, detorubicin, 6-diazo-5-oxo-L-norleucine, doxorubicin, morpholino-doxorubicin, cyanomorpholino-doxorubicin, 2-pyrrolino-doxorubicin, liposomal doxorubicin, deoxydoxorubicin, epirubicin, Esorubicin, marcellomycin, mitomycin C, mycophenolic acid, nogalamycin, olivomycins, peplomycin, potfiromycin, puromycin, quelamycin, rodorubicin, streptomigrin, streptozocin, tubercidin, ubenimex, zinostatin, zorubicin, 5-fluorouracil, denopterin, methotrexate, pteropterin, trimetrexate, fludarabine, 6-mercaptopurine, thiamiprine, tiguanine, ancitabine, azacitidine, 6-azauridine, carmofur, cytarabine, dideoxyuridine, doxifluridine, enocitabine, floxuridine, calusterone, dromostanolone, propionate, epitiostanol, mepitiostane, testolactone, Aminoglutethimide, mitotane, trilostane, folinic acid, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, etoglucid, gallium nitrate, hydroxyurea, lentinan, lonidainine, maytansine, ansamitocins, mitoguazone, mitoxantrone, mopidanmol, nitraerine, pentostatin, phenamet, pirarubicin, losoxantrone, 2-ethylhydrazide, procarbazine, polysaccharidek, razoxane, rhizoxin, sizofiran, spirogermanium, tenuazonic acid, triaziquone, 2,2',2"-trichlorotriethylamine, T-2 toxin, verracurin A, roridin A, anguidine, urethane, vindesine, dacarbazine, mannomustine, mitobronitol, mitolactol, pipobroman, gacytosine, arabinoside, cyclophosphamide, thiotepa, paclitaxel, albumin-engineered nanoparticle formulation of paclitaxel, docetaxel, chlorambucil, gemcitabine, 6-thioguanine, mercaptopurine, cisplatin, carboplatin, vinblastine, platinum, etoposide, ifosfamide, mitoxantrone, vincristine, vinorelbine, novantrone, teniposide, edatrexate, daunomycin, aminopterin, xeloda, ibandronate, CPT-11, topoisomerase inhibitor RFS 2000, difluoromethylornithine, retinoic acid, capecitabine, or a pharmaceutically acceptable salt, solvate or acid thereof;

(b) monokines, lymphokines, traditional polypeptide hormones, parathyroid hormone, thyroxine, relaxin, prorelaxin, glycoprotein hormone, follicle-stimulating hormone, thyroid-stimulating hormone, luteinizing hormone, hepatic growth factor fibroblast growth factor, prolactin, placental lactogen, tumor necrosis factor, tumor necrosis factor-α, tumor necrosis factor-β, mullerian inhibiting substance, mouse gonadotropin-associated peptide, Inhibin, activin, vascular endothelial growth factor, thrombopoietin, erythropoietin, osteoinductive factor, interferon, interferon-α, interferon-β, interferon-γ, colony stimulating factor (CSF), macrophage-CSF, granulocyte-macrophage-CSF, granulocyte-CSF, interleukin (IL), IL-1, IL-1α, IL-2, IL-3, IL-4, IL-5, IL-6, IL-7, IL-8, IL-9, IL-10, IL-11, IL-12, tumor necrosis factor, polypeptide factor, LIF, kit ligand ligand), or a combination thereof;

(c) diphtheria toxin, botulinum toxin, tetanus toxin, decentri toxin, cholera toxin, amanitin, α-amanitin, pyrrolobenzodiazepine, pyrrolobenzodiazepine derivatives, indolinobenzodiazepine, pyridinobenzodiazepine, tetrodotoxin, brevetoxin, ciguatoxin, ricin, AM toxin, auristatin, tubulisin, geldanamycin, maytansinoid, calicheamycin, daunomycin, doxorubicin, methotrexate, vindesine, SG2285, dolastatin, dolastatin analog, auristatin, cryptophycin, camptothecin, rhizoxin, rhizoxin derivatives, CC-1065, CC-1065, duocarmycin, enediyne antibiotic, esperamicin, epothilone, toxoid, or a combination thereof;

(d) an immunomodulatory compound, an anticancer agent, an anti-viral agent, an antibacterial agent, an anti-fungal agent, and an anti-parasitic agent, or a combination thereof;

(e) tamoxifen, raloxifene, droloxifene, 4-hydroxytamoxifen, trioxifene, keoxifene, LY117018, onapristone, or toremifene;

(f) 4(5)-imidazole, aminoglutethimide, megestrol acetate, exemestane, letrozole, anastrozole; or

(g) flutamide, nilutamide, bicalutamide, leuprolide, goserelin, or troxacitabine.

The above antibody or functional fragment thereof not only binds to ITGA3 or VLA3, but also has the advantage of being internalized into cells and effectively delivering the conjugated drug into cells when applied as an antibody-drug conjugate (ADC).

Another aspect provides a chimeric antigen receptor (CAR) protein comprising the antibody or functional fragment thereof.

Another aspect provides immune cells comprising the chimeric antigen receptor protein.

As used herein, the term "chimeric antigen receptor (CAR)" refers to a non-naturally occurring receptor capable of conferring specificity for a specific antigen to immune effector cells. Typically, the CAR refers to a receptor used to transfer the specificity of a monoclonal antibody to T cells. A CAR typically consists of an extracellular domain (Ectodomain), a transmembrane domain, and an intracellular domain (Endodomain).

The above extracellular domain comprises the above antibody or functional fragment thereof, which contains an antigen recognition region. The antibody used in the CAR is preferably in the form of an antibody fragment, and may be in the form of scFv, but is not particularly limited thereto.

In addition, the transmembrane domain of the CAR, which is in a form linked to the extracellular domain, may be derived from a natural or synthetic source. If derived from a naturally occurring source, it may be derived from a membrane-bound or membrane-permeable protein, and may be a portion derived from a membrane-permeable region of various proteins such as the alpha, beta or zeta chain of the T cell receptor, CD28, CD3 epsilon, CD45, CD4, CD5, CDS, CD9, CD16, CD22, CD33, CD37, CD64, CD80, CD86, CD134, CD137, CD154 or CD8, but is not limited thereto. The sequence of such a transmembrane domain may be obtained from a document known in the art that discloses a membrane-permeable region portion of a membrane-permeable protein.

In the above CAR, the intracellular signaling domain exists inside the cell in a form connected to the transmembrane domain. The intracellular signaling domain of the present invention is a region that primarily generates and/or transmits a signal that causes cell activation when an antigen binds to the antigen binding site of the CAR (i.e., the antibody of the present invention or functional fragment thereof).

The above "cell" is not particularly limited in type, but may preferably be an immune cell (immune effector cell). The immune cell is not particularly limited in type as long as it is a cell known in the art to be involved in the body's immune function, but includes, for example, T cells, NK (Natural Killer) cells, NKT (Natural Killer T) cells, monocytes, macrophages, or dendritic cells, and also includes precursor cells thereof.

As used herein, "antibody" means any isotype of intact immunoglobulin, an intact antibody, or antigen-binding fragment thereof, capable of binding to a target antigen. Examples include chimeric, humanized, fully human, and bispecific antibodies, or antigen-binding fragments thereof. The antibody itself is a type of antigen-binding protein. The intact antibody typically comprises at least two full-length heavy chains and two full-length light chains, although in some cases, antibodies found naturally in camelids may comprise only heavy chains. The antibody or antigen-binding fragment thereof may be derived from a single source or a chimera. The chimeric antibody comprises portions derived from two different types of antibodies and is described in more detail below. The antibody or antigen-binding fragment thereof may be produced by hybridoma, recombinant DNA technology, or enzymatic or chemical cleavage of an intact antibody. Unless otherwise stated, the term antibody in this specification includes derivatives, variants, fragments and mutants thereof, examples of which are described below.

The term "antibody fragment" or "antigen-binding fragment" as used herein is a portion of an antibody, such as scFv-Fc, F(ab')2, F(ab)2, Fab', Fab, Fv, scFv, etc. Regardless of structure, an antibody fragment binds to the same antigen recognized by an intact antibody. The term "antibody fragment" includes aptamers, enantiomers, scFvs, dAbs, diabodies, maxibodies, etc. The term "antibody fragment" also includes any synthetic or genetically engineered protein that functions similarly to an antibody by binding to a specific antigen and forming a complex.

"Single-chain variable fragment" or "v" refers to a fusion protein of the variable regions of the heavy (VH) and light (VL) chains of an immunoglobulin. In one embodiment, the regions may be linked by a short linker peptide of 10 to about 25 amino acids. The linker may be glycine-rich for flexibility, and may also be serine- or threonine-rich for solubility, and may link the N-terminus of the VH to the C-terminus of the VL, or vice versa. Furthermore, in one embodiment, the linker may comprise mutations. The protein retains the specificity of the native immunoglobulin even with the removal of a constant region and the introduction of a linker. scFv molecules are well known in the art and are described, for example, in U.S. Patent No. 5,892,019.

The term antibody encompasses a diverse and broad class of biochemically distinguishable polypeptides. Those skilled in the art will recognize that heavy chains are classified as gamma, mu, alpha, delta, or epsilon ($\gamma$, $\mu$, $\alpha$, $\delta$, $\epsilon$), and some of them include subclasses (e.g., $\gamma$l-$\gamma$). It is the properties of these chains that determine the "class" of the antibody, as IgG, IgM, IgA, IgG, or IgE, respectively. The immunoglobulin subclasses (isotypes), e.g., IgG1, IgG2, IgG3, IgG4, IgG5, etc., are well characterized and are known to confer functional specificity. Modified versions of each of these classes and isotypes are readily distinguishable to those skilled in the art in view of the present invention. With respect to IgG, a standard immunoglobulin molecule comprises two identical light chain polypeptides with a molecular weight of approximately 23,000 daltons, and two identical heavy chain polypeptides with a molecular weight of 53,000-70,000. The four chains are typically linked in a "Y" configuration via disulfide bonds, with the light chain starting at the entrance of the "Y" and continuing through the variable region to bracket the heavy chain.

In one embodiment, the antibody, antigen-binding polypeptide, variant, or derivative thereof includes, but is not limited to, polyclonal, monoclonal, multispecific, human, humanized, primatized, or chimeric antibodies, single-chain antibodies, epitope-binding fragments such as Fab, Fab' and F(ab')2, Fd, Fvs, single-chain Fv (scFv), single-chain antibodies, disulfide-linked Fv (sdFv), fragments comprising VK or VH domains, fragments produced by Fab expression libraries, and anti-idiotypic (anti-Id) antibodies. An immunoglobulin or antibody molecule according to one embodiment can be any type or subclass of immunoglobulin molecule (e.g., IgG1, IgG2, IgG3, IgG4, IgA1, and IgA2).

Light chains are classified as kappa or lambda (K, $\lambda$). Each heavy chain classification can also be combined with a kappa or lambda light chain. Generally, the light and heavy chains are covalently bonded to each other, and the "tail" portions of the two heavy chains are linked to each other either by a covalent disulfide bond or, when the immunoglobulin is produced by a hybridoma, B cell, or genetically engineered host cell, by a non-covalent bond. In the heavy chain, the amino acid sequence runs from the N-terminus of the forked end of the Y configuration to the C-terminus of the bottom of each chain.

Both light and heavy chains are divided into regions of structural and functional homology. The terms "constant" and "variable" are used functionally. In this regard, it can be seen that the variable domains of both the light (VL) and heavy (VH) chain portions determine antigen recognition and specificity. Conversely, the constant domains of the light (CL) and heavy chains (CH1, CH2, or CH3) confer important biological properties such as secretion, transplacental mobility, binding to Fc receptors, complement binding, etc. Conventionally, the numbering of constant region domains increases with distance from the antigen-binding site or amino acid terminus of the antibody. The N-terminal portion is the variable region and the C-terminal portion is the constant region. The CH3 and CL domains actually comprise the carboxy-terminus of the heavy and light chains, respectively.

As indicated above, the variable region allows the antibody to selectively recognize and specifically bind to an epitope on an antigen. That is, the VL domain and VH domain, or a subset of the complementary determining regions (CDRs), of the antibody combine to form the variable region, which defines a three-dimensional antigen-binding site. This quaternary structure forms the antigen-binding site present at each N-terminus of Y. More specifically, the antigen-binding site is defined by three CDRs (i.e., CDR-H1, CDR-H2, CDR-H3, CDR-L1, CDR-L2, and CDR-L3) on each of the VH and VL chains. In some cases, for example, certain immunoglobulin molecules derived from camelid species or engineered based on camelid immunoglobulins, complete immunoglobulin molecules may consist solely of heavy chains, without light chains. See, for example, Hamers-Casterman et al., Nature 363: 446-448 (1993).

In naturally occurring antibodies, the six "complementary determining regions" or "CDRs" present in each antigen-binding domain are short, non-contiguous sequences of amino acids that are specifically positioned to form the antigen-binding domain because the antibody adopts a three-dimensional configuration in an aqueous environment. The remaining amino acids within the antigen-binding domain, called the "framework" region, exhibit less intermolecular variability. The framework region predominantly adopts the β-sheet conformation, and the CDRs connect these β-sheet structures and, in some cases, form loops that constitute part of them. Thus, the framework region functions to form a scaffold that positions the CDRs in the correct orientation by non-covalent interchain interactions. The antigen-binding domain formed by the positioned CDRs defines a surface complementary to an epitope on an immunoreactive antigen. This complementary surface facilitates non-covalent binding of the antibody to its cognate epitope. The amino acids comprising the CDR and framework regions are each precisely defined and can be readily identified by one skilled in the art for any given heavy or light chain variable region (see www.bioinf.org.uk: Dr. Andrew C.R. Martin's Group; "Sequences of Proteins of Immunological Interest," Kabat, E., et al., U.S. Department of Health and Human Services, (1983); and Chothia and Lesk, J. Mol. Biol., 196: 901-917 (1987)).

Where two or more definitions of a term are used and/or accepted in the art, the definition of said term used herein is intended to encompass all such meanings unless explicitly stated otherwise. A specific example is the use of the term "complementary determining region" ("CDR") to describe the non-adjacent antigen-combining sites found within the variable regions of both heavy chain and light chain polypeptides. A number of methods can be used to define a CDR. Current technology employs various numbering systems utilizing different definitions of CDR length and position. For example, the Kabat numbering system is based on sequence alignments and predicts CDRs using the "variability parameter" (the number of different amino acids at a given position divided by the frequency of the most abundant amino acid at that position) for a given amino acid position. In contrast, the Chothia numbering system is a structure-based numbering system in which antibody crystal structures are aligned when loop structures are defined as CDRs. The Martin numbering system focuses on structural alignment of different framework regions of unusual length. The IMGT numbering system is a standardized numbering system based on alignment of sequences from a complete reference gene database containing the entire immunoglobulin superfamily. Honneger's numbering system (AHo's) is based on the structural alignment of the 3D structures of the variable regions and uses structurally conserved Cα positions to infer framework and CDR lengths.

When numbered according to the Kabat numbering system, residues 24-34 (L1), 50-56 (L2), and 89-97 (L3) in VL, and approximately 31-35 (H1), 50-65 (H2), and 95-102 (H3) in VH (Kabat et al. Sequences of Proteins of Immunological Interest, 5th Ed. Public Health Service, National Institutes of Health, Bethesda, MD, 1991). When numbered according to the Chothia numbering system, these correspond to residues 24-34 (L1), 50-56 (L2), and 89-97 (L3) in VL, and 26-32 (H1), 52-56 (H2), and 95-101 (H3) in VH (Chothia and Lesk. J. Mol. Biol. 1987, 196, 901-917). When numbered according to the IMGT numbering system, residues 27-38 (L1), 56-65 (L2), and 105-120 (L3) in VL, and 27-38 (H1), 56-65 (H2), and 105-120 (H3) in VH (Lefranc et al. Nucl. Acids Res. 1999, 27, 209-212, Ruiz et al. Nucl. Acids Res. 2000, 28, 219-221). When numbered according to Honneger's numbering system (AHo's), the positions are 28, 36 (L1), 63, 74-75 (L2), and 123 (L3) in VL and 28, 36 (H1), 63, 74-75 (H2), and 123 (H3) in VH [Honneger and Plunkthun (Mol. Biol. 2001, 309, 657-670)].

Those skilled in the art will recognize that the definition of a CDR may vary depending on the method used. Any method for defining a CDR is considered in conjunction with the sequences disclosed herein.

The antibodies disclosed herein may be derived from any animal origin, including birds and mammals. For example, the antibodies may be human, rodent, donkey, rabbit, goat, guinea pig, camel, llama, horse, or chicken antibodies.

The term "heavy chain constant region" as used herein includes amino acid sequences derived from immunoglobulin heavy chains. A polypeptide containing a heavy chain constant region comprises at least one of the following: a CH1 domain, a hinge domain (e.g., an upper, middle, and/or lower hinge region), a CH2 domain, a CH3 domain, or a variant or fragment thereof. For example, an antigen-binding polypeptide for use in the present invention can include: a polypeptide chain containing a CH1 domain; a polypeptide chain containing a CH1 domain, at least a portion of a hinge domain, and a CH2 domain; a polypeptide chain containing a CH1 domain and a CH3 domain; a polypeptide chain containing a CH1 domain, at least a portion of a hinge domain, and a CH3 domain; or a polypeptide chain comprising a CH1 domain, at least a portion of a hinge domain, a CH2 domain, and a CH3 domain. Those skilled in the art will understand that the heavy chain constant regions may be modified to differ in amino acid sequence from naturally occurring immunoglobulin molecules.

The heavy chain constant region of the antibody disclosed herein may be derived from different immunoglobulin molecules. For example, the heavy chain constant region of the polypeptide may include a CH1 domain derived from an IgG1 molecule and a hinge region derived from an IgG3 molecule. In another example, the heavy chain constant region may include a hinge region derived in part from an IgG1 molecule and in part from an IgG3 molecule. In yet another example, the heavy chain portion may include a chimeric hinge derived in part from an IgG1 molecule and in part from an IgG4 molecule.

The term "light chain constant region" as used herein includes an amino acid sequence derived from an antibody light chain. For example, the light chain constant region includes at least one of a constant kappa domain or a constant lambda domain.

The subunit structures and three-dimensional conformation of the constant regions of various immunoglobulin classes are well known. As used herein, the term "domain" includes the amino-terminal variable domain of an immunoglobulin heavy chain, and the term "domain" includes the first (most amino-acid-terminal) constant region domain of an immunoglobulin heavy chain. The CH1 domain is adjacent to the VH domain and is located at the amino terminus relative to the immunoglobulin heavy chain molecule and hinge region.

The term "domain" as used herein includes, for example, the portion of a heavy chain molecule extending approximately from residue 244 to residue 360 using the conventional numbering scheme (Kabat numbering system: residues 244 to 360; and EU numbering system: residues 231-340). (see Kabat et al., U.S. Dept. of Health and Human Services, "Sequences of Proteins of Immunological Interest" (1983)). The CH2 domain is unique in that it does not form a close pair with another domain. Instead, two N-linked branched carbohydrate chains are interposed between the two CH2 domains of an intact, native IgG molecule. Furthermore, it is well documented that the CH3 domain extends from the CH2 domain to the C-terminus of the IgG molecule and contains approximately 108 residues.

The term "hinge region" as used herein encompasses the portion of a heavy chain molecule that links the CH1 domain to the CH2 domain. This hinge region comprises approximately 25 residues and is flexible, thus allowing the two N-terminal antigen-binding domains to move independently. The hinge region can be subdivided into three distinct domains: the upper, middle, and lower hinge domains (Roux et al., J. Immunol 161: 4083 (1998)).

The term "disulfide bond" as used herein encompasses the covalent bond formed between two sulfur atoms. The amino acid cysteine comprises a thiol group capable of forming a disulfide bond or bridge with a second thiol group. In most naturally occurring IgG molecules, the CH1 and CL regions are linked by a disulfide bond, and the two heavy chains are linked by two disulfide bonds at positions corresponding to positions 239 and 242 (positions 226 or 229, EU numbering system) in Kabat numbering system.

Here, the term "antigen-binding region or site" refers to a protein or a portion of a protein that specifically binds to a particular antigen. For example, it refers to a portion of an antibody that comprises amino acid residues that interact with an antigen and provide the antibody with specificity and affinity for the antigen. This antigen-binding region typically includes one or more "complementary determining regions (CDRs)." Certain antigen-binding regions also include one or more "framework (FR)" regions. The framework regions help maintain the proper conformation of these CDRs, thereby facilitating binding between the antigen-binding region and the antigen.

The "Fc" region herein comprises two heavy chain fragments comprising the CH2 and CH3 domains of an antibody. These two heavy chain fragments are associated by two or more disulfide bonds and hydrophobic interactions of the CH3 domain.

The Fc domain may be an IgG Fc domain, for example, an Fc domain of IgG1, IgG2, IgG3, IgG4 or a combination thereof, and the Fc domain of the immunoglobulin may be a wild-type Fc domain as well as an Fc domain variant. In addition, the term "Fc domain variant" as used herein may have a different glycosylation pattern from that of a wild-type Fc domain, or may have increased glycosylation compared to a wild-type Fc domain, decreased glycosylation compared to a wild-type Fc domain, or a deglycosylated form. An aglycosylated Fc domain is also included. The Fc domain or variant may have a controlled number of sialic acid, fucosylation, or glycosylation through culture conditions or genetic manipulation of the host.

The above Fc plays a role in enhancing the stability of the antibody, and may have effects such as extending the half-life (e.g., half-life in the body) and/or reducing renal filtration. In one specific example, the Fc region of the immunoglobulin may be selected from the Fc of IgG1 and the Fc of IgG4. The Fc region of IgG1 may include the CH2 domain, the CH3 domain, or the CH2+CH3 domain of IgG1, and may or may not include the hinge region of IgG1 at the N-terminus. The Fc region of IgG4 may include the CH2 domain, the CH3 domain, or the CH2+CH3 domain of IgG4, and may or may not include the hinge region of IgG4 at the N-terminus.

IgG1 may be derived from a primate such as a human or a rodent such as a mouse or rat, and may be, for example, human IgG1 (UniProtKB P01857). IgG4 may be derived from a primate such as a human or a rodent such as a mouse or rat, and may be, for example, human IgG4 (UniprotKB P01861).

**EP 4 703 380 A1**

When the Fc region of an immunoglobulin is used to extend the (in vivo) half-life of a protein linked to its N-terminus or C-terminus, it is important to minimize any effector functions of the Fc in order to reduce adverse effects caused by the immunoglobulin Fc region. In this respect, the human IgG4 Fc region is advantageous because it has lower binding affinity to FcγR and complement factors compared to other IgG subtypes. In addition, the human IgG4 Fc region may exhibit reduced effector function due to amino acid substitutions. The amino acid substitutions in the human IgG4 Fc region to reduce the above effector function may include one or more of the following: substitution of phenylalanine at position 234 of human IgG4 (UniprotKB P01861) with alanine, and substitution of leucine at position 235 with alanine (included within the CH2-CH3 domain region of IgG4 Fc; the amino acid residue numbers follow EU numbering [Kabat, E.A. et al. (1991) Sequences of Proteins of Immunological Interest, 5th ed., U.S. Dept. of Health and Human Services, Bethesda, MD, NIH Publication no. 91-3242]). Additionally, the Fc region of IgG4 may include an amino acid substitution that stabilizes heavy chain dimer formation and prevents the formation of half-IgG4 Fc chains. Such amino acid substitutions may include substitution of serine at the 228th amino acid residue (according to EU numbering; included within the hinge region) of the Fc region of human IgG4 (UniprotKB P01861) with proline.

The term "antigen" or "immunogen" as used herein refers to a molecule or a portion of a molecule to which an antigen-binding protein (e.g., an antibody or an immunologically functional antigen-binding fragment thereof) can bind and which can be used to produce an antibody capable of binding to an antigen in an animal. The antigen may comprise one or more epitopes capable of interacting with different antibodies or fragments thereof. In one embodiment, the antigen is a membrane protein of a cancer stem cell or a cancer cell, and specifically may be ITGA3 (Integrin Subunit Alpha 3) or VLA3 (Integrin alpha 3 beta 1).

As used herein, the term "epitope" refers to a portion of a molecule that is bound to or recognized by an antigen-binding protein or antibody, and includes, for example, any determinant capable of specifically binding to an antigen-binding protein, such as an antibody or T-cell receptor. The epitope may be contiguous or discontinuous. For example, in a polypeptide sequence, they may be amino acid residues bound by a single antigen-binding protein from a molecular perspective, such as a conformational epitope, even if they are not contiguous with each other, but may be separated. In one embodiment, the epitope may be mimetic in that it contains a three-dimensional structure similar to the epitope used for antibody production, but may not contain residues found in the epitope or may contain only some of the residues. Typically, the epitope is a protein, but may also be another type of substance, such as a nucleic acid. The above epitope-determining factor may be a chemically active group formed on the surface by a molecule such as an amino acid, a sugar side chain, a phosphoryl group, or a sulfonyl group, or may have specific three-dimensional structural characteristics and/or specific charge characteristics. Generally, an antibody specific to a specific target antigen recognizes an epitope of the target antigen present in a protein and/or polymer complex.

As used herein, the term "amino acid" includes the general meaning understood in the art. The 20 naturally occurring amino acids and their abbreviations are as commonly used in the art (Immunology-A Synthesis, 2nd Edition, E. S. Golub and D. R. Green, eds., Sinauer Associates: Sunderland, Mass. 1991). The amino acids include the typical amino acids, stereoisomers of the typical 20 amino acids (D-amino acids), non-natural amino acids, such as α-,α-disubstituted amino acids, N-alkyl amino acids, and other non-typical amino acids. Examples of non-canonical amino acids include 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the mark of polypeptide used herein, as is generally used in the art, the left side of the sequence represents the amino terminus and the right side represents the carboxy terminus.

As used herein, "polypeptide" or "protein" refers to a polymer of amino acid residues and is used interchangeably herein. This also includes polymers of naturally occurring amino acid residues as well as polymers of their analogues or mimetics. Furthermore, the polypeptide or protein may include modifications, such as the addition of carbohydrates for phosphorylation or glycosylation. Moreover, the aforementioned polypeptide or protein may be produced in recombinant or naturally occurring cells. Furthermore, the polypeptide or protein may include a wild-type sequence or those in which portions of the amino acid sequence have been deleted, added, and/or substituted. Furthermore, the polypeptide or protein may be an antibody, or an antigen-binding fragment, or a polypeptide in which one or more amino acids have been deleted, added, and/or substituted in the sequence. The term "polypeptide fragment" refers to a polypeptide having an amino-terminal deletion, a carboxyl-terminal deletion, and/or an internal deletion, compared to the full-length protein. The fragment may also comprise modified amino acids compared to the full-length protein. In one embodiment, the fragment may be about 5 to 500 amino acids in length, for example, at least 5, 6, 8, 10, 14, 20, 50, 70, 100, 110, 150, 200, 250, 300, 350, 400, 450, 500, 550, 600, 650, 700, 750, 800, 850 or more amino acids in length. In view of the purposes of the present invention, the useful polypeptide fragment comprises an immunologically functional fragment of an antibody containing an antigen-binding domain. For antibodies binding to a cancer stem cell or cancer cell membrane protein according to one embodiment, such useful fragments include, but are not limited to, CDR sequences comprising one, two or three heavy or light chains, or all or part of an antibody chain containing the variable or constant regions of the heavy or light chain.

As used herein, the term "isolated polypeptide, antibody, or protein" means that they are free of other proteins normally found with them, and at least about 50% or more of the lipids, carbohydrates, and polynucleotides naturally associated with them have been removed. Typically, the isolated protein, polypeptide, or antibody comprises about 5% or more, about 10% or more, about 25% or more, or about 50% or more in a specific composition. This polypeptide may be encoded by genomic DNA, cDNA, mRNA, or other RNA of synthetic origin, or by any combination thereof. In particular, the isolated protein, polypeptide, or antibody is substantially free of contaminants from other proteins or other polypeptides that would interfere with its application in therapeutic, diagnostic, and preventive research or other uses.

The antibodies and/or antigen-binding fragments thereof disclosed herein may include "variants", which may refer to polypeptides in which one or more amino acid residues are inserted, deleted, added and/or substituted from the polypeptide sequence. Therefore, provided that the desired biological activity and/or structure of the antibody and/or antigen-binding fragment is maintained, they may be linked to other polypeptides to form fusion polypeptides. Additionally, the variants may be modified by protein enzyme cleavage, phosphorylation, and/or other post-translational modifications, but may include modifications that maintain the biological activity of the antibodies and/or antigen-binding fragments disclosed herein, such as binding and specificity for ITGA3 or VLA3. The above variants may have sequence identity of 80% or more, 85% or more, 90% or more, 95% or more, 96% or more, 97% or more, 98% or more, or 99% or more with respect to the sequence of the antibody or antigen-binding fragment thereof disclosed herein, for example, may have about 99%, 98%, 97%, 96%, 95%, 94%, 93%, 92%, 91%, 90%, 89%, 88%, 87%, 86%, 85%, 84%, 83%, 82%, 81%, or 80% sequence identity. The percent identity (%) or homology can be calculated as 100 X [(identical position)/min(TGA, TGB)], where TGA, TGB are the sum of the number of residues in the comparison sequences A and B. Comparison and internal gap positions (Russell et al., J. Mol Biol., 244: 332-350 (1994).

As used herein, the term "conservative amino acid substitution" refers to a substitution that does not substantially affect the activity or antigenicity of the polypeptide. The polypeptide may contain one or more conservative substitutions.

As used herein, a "derivative" of a polypeptide refers to a polypeptide that has been chemically modified at one or more residues through conjugation with a different chemical moiety, distinct from insertion, deletion, addition, or substitution variants.

As used herein, "identity" means the sequence similarity of two or more polypeptides or two or more polynucleotides, as determined by aligning and comparing them. The identity between these sequences is generally expressed as a "percent identity," which refers to the proportion of identical amino acids or nucleotides between the molecules being compared, and is calculated based on the size of the smallest molecule among the molecules being compared.

One aspect of the present invention relates to antibodies that bind to cancer stem cells or cancer cell membrane proteins, specifically recombinant antibodies that specifically bind to ITGA3 or VLA3 or their antigen-binding fragments. In this aspect, "recombinant protein" refers to a protein produced using recombinant technology, i.e., through the expression of a recombinant nucleic acid according to one embodiment. The methods and techniques for producing recombinant proteins are well known in the art.

As used herein, the term "affinity" or "binding affinity" refers to the strength of the interaction between an antibody or antigen-binding fragment thereof and an antigen, and is determined by the characteristics of the antigen, such as the size, shape, and/or charge of the antigen, and by the CDR sequences of the antibody or antigen-binding fragment. Methods for determining the affinity are known in the art.

The antibody or antigen-binding fragment thereof is said to "specifically bind" to a target, such as an antigen, when the dissociation constant (KD) is $10^{-6}$ M or less. The antibody specifically binds to a target with "high affinity" when its KD is $1 \times 10^{-8}$ M or lower.

As demonstrated in the Examples, the antibodies specific to the cancer stem cells or cancer cell membrane proteins disclosed herein, specifically anti-ITGA3 or anti-VLA3 antibodies, exhibit ITGA3 or VLA3 binding capability, binding capability to ITGA3 or VLA3 expressed on the cell surface, and high ITGA3 or VLA3 binding affinity. Furthermore, as demonstrated in the Examples, the anti-ITGA3 or anti-VLA3 antibodies disclosed herein exhibit an *in vivo* antitumor effects.

These anti-ITGA3 or anti-VLA3 antibodies may be useful for therapeutic purposes, such as the treatment of various types of cancer, and may also be used for diagnostic and prognostic purposes.

Another aspect of the present invention provides a pharmaceutical composition for treating or preventing cancer, comprising the antibody or a functional fragment thereof.

Another aspect of the present invention provides a method for treating or preventing cancer, comprising a step of administering an effective amount of the antibody or a functional fragment thereof to a subject in need thereof.

The cancers mentioned above may include, but are not limited to, blood cancer, lung cancer, stomach cancer, liver cancer, bone cancer, pancreatic cancer, skin cancer, head and neck cancer, skin melanoma, uterine cancer, ovarian cancer, rectal cancer, brain cancer, colorectal cancer, colon cancer, breast cancer, triple negative breast cancer, uterine sarcoma, fallopian tube carcinoma, endometrial carcinoma, uterine cervical carcinoma, vaginal carcinoma, vulva carcinoma, esophageal cancer, laryngeal cancer, small-intestine cancer, thyroid cancer, parathyroid cancer, soft-tissue sarcoma, urethral cancer, penile cancer, prostate cancer, chronic or acute leukemia, solid tumors in juvenile stage, differentiated lymphoma, bladder cancer, oral cavity carcinoma, renal cancer, renal cell carcinoma, renal pelvic carcinoma, primary central nervous system lymphoma, spinal axis tumors, glioma, brain stem glioma, and pituitary adenoma.

The above cancer may include solid cancer and non-solid cancer.

As used herein, the term "therapeutic agent" or "pharmaceutical composition" refers to a molecule or composition administered to a subject for targeted therapeutic effects. The subject includes non-human mammals, e.g., primates or humans. Examples of such therapeutic agents include proteins including peptides and polypeptides, nucleic acids, antibodies, or small molecular compounds. In another aspect, the therapeutic agent may be used as a treatment for a disease, such as cancer, by binding to an antibody or a functional fragment thereof.

The term "treating" as used herein means the alleviation or cure of injuries, illnesses, symptoms of illness, or disease state involving any objective or subjective parameter. It includes the reduction or alleviation of an injury, disease, or pathological condition; enabling the patient to better tolerate the symptoms of an injury, disease, or pathological condition; slowing the rate of worsening of the injury, disease, or the symptoms of pathological condition; or improving the patient's mental or physical quality of life. The treatment or improvement of an injury, disease, or symptoms of pathological condition may be determined based on the results of a physical examination, various disease-related indicator tests, and imaging tests.

As used herein, the term "effective amount (dose)" means an amount sufficient to reduce the severity and/or incidence of a disease, eliminate the underlying cause of the disease, prevent the occurrence of symptoms caused by the underlying cause of the disease, and/or improve or correct damage caused by the disease. In one embodiment, the effective dose is either a therapeutic effective dose or a prophylactic effective dose. The "therapeutic or pharmaceutically effective dose" is an amount sufficient to treat, prevent, delay, or reverse the progression of symptoms or conditions associated with a disease. A "prophylactic effective dose" is a dose required to prevent or delay the onset or recurrence of a disease or its symptoms, and to reduce their severity. The full therapeutic or prophylactic effect may be achieved by multiple doses rather than a single dose. Therefore, the therapeutic or prophylactic effective dose may be delivered by one or more administrations. The effective amount may be appropriately selected by a person skilled in the art based on the target cells or organisms. It may be determined based on factors including the severity of the disease, the patient's age, weight, health, gender, the patient's sensitivity to the drug, the time of administration, the route of administration, the rate of excretion, the duration of treatment, the medications used in combination with or concurrently with the composition, and other factors well known in the medical field.

The present invention provides a method for treating a cancer patient by administering a pharmaceutical composition comprising a therapeutically effective amount of an antibody, or antibody conjugate, or a pharmaceutically acceptable salt or solvate thereof, and a pharmaceutically acceptable diluent, carrier, solubilizer, emulsifier, preservative, and/or excipients. The term "patient" includes a human patient.

The pharmaceutical composition may include a pharmaceutically acceptable carrier. The term "carrier" is used to mean an excipient, diluent, or adjuvant. The carrier may be selected from the group consisting of, for example, lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinyl pyrrolidone, water, saline, a buffer such as PBS, methylhydroxybenzoate, propylhydroxybenzoate, talc, magnesium stearate, and mineral oil. The composition may include fillers, anticoagulants, lubricants, wetting agents, flavoring agents, emulsifiers, preservatives, or combinations thereof.

The pharmaceutical composition described above may be prepared in any formulation by conventional methods. For example, the composition may be formulated as an oral dosage form (e.g., powder, tablet, capsule, syrup, pill, or granule) or a parenteral dosage form (e.g., injection). Furthermore, the composition may be prepared as a systemic dosage form or a topical dosage form.

Another aspect of the present invention provides a polynucleotide encoding the antibody or a functional fragment thereof.

Another aspect of the present invention provides a recombinant vector containing the polynucleotide.

Another aspect of the present invention provides a recombinant cell comprising the above vector.

As used herein, "polynucleotide" or "nucleic acid" includes a single- or double-stranded nucleotide polymers. The nucleotides comprising such a polynucleotide may be ribonucleotides or deoxyribonucleotides, or modified forms thereof.

As used herein, the term "isolated nucleic acid molecule" means DNA, RNA, mRNA, or cDNA of genomic origin, or a combination thereof, wherein all or part thereof is associated with a polynucleotide not found in nature or not occurring in nature, and is linked to said polynucleotide. For the purposes of the present invention, the nucleic acid molecule comprising a specific nucleic acid sequence does not comprise an intact chromosome. Instead, the isolated nucleic acid molecule containing the specific nucleic acid sequence may comprise at least several additional protein-coding sequences in addition to its specific sequence, or may further comprise regulatory sequences and/or vectors for the expression of the specific nucleic acid sequence.

As used herein, the term "regulatory sequence" refers to a polynucleotide sequence operably linked and capable of affecting the expression and processing of a coding sequence. The characteristics of a regulatory sequence may be influenced by the type of host. For example, a regulatory sequence applicable to prokaryotic cells may include promoters, sometimes operators, ribosome binding sites, and transcription termination sequences. In eukaryotic cells, the regulatory sequence may include a promoter containing multiple recognition sites, a transcription enhancer, a polyadenylation sequence, and a transcription termination sequence. The regulatory sequence may additionally include a reader sequence and/or a fusion partner sequence.

As used herein, "vector" means any molecule used to deliver a nucleic acid molecule encoding proteins to host cells, including, for example, nucleic acids, plasmids, bacteriophages, or viruses.

As used herein, the term "expression vector" or "recombinant vector" refers to a vector suitable for transformation into host cells, and includes a nucleic acid sequence that is operably linked to an expression vector and controls the expression of a heterologous sequence encoding a target protein. This expression vector may also be operably linked to a coding sequence and may contain sequences involved in transcription, translation, and, if introns are present, RNA splicing.

As used herein, the term "operably (or operatively) connected (or linked)" means that the nucleic acid sequence to be linked is positioned so that it can perform the targeting function under appropriate conditions. For example, in a vector containing a coding sequence and a regulatory sequence, when transcription of the coding sequence is affected by the regulatory sequence under appropriate conditions, the vector is operably linked.

As used herein, "host cell" means a cell capable of expressing a target gene that is or will be transformed by a target nucleic acid sequence. The above term includes the progeny of the host cells that express the target gene, regardless of the identity, morphology, and genetic composition of the host cells.

As used herein, "transduction" generally refers to the transfer of nucleic acid from one bacterium to another, typically by a bacteriophage. For example, this includes the transfer of nucleic acid into a eukaryotic cell using non-replicating retroviruses.

As used herein, "transfection" refers to the acquisition of foreign or exogenous DNA by a cell, wherein the DNA is introduced into the cell through the cell membrane. This can be accomplished by methods known in the art, such as those described in Sambrook et al., Molecular Cloning: A Laboratory Manual, 4th ed., Cold Spring Harbor Laboratory Press, Cold Spring Harbor, N.Y. (2012), and Ausubel et al., Current Protocols in Molecular Biology, Greene Publishing Associates.

As used herein, "transformation" refers to an alteration in the genetic characteristics of a cell that has been modified to contain new DNA or RNA. For example, cells may be transformed by introducing new genetic material through transduction, transfection, or other techniques, thereby altering its genetic characteristics. Transformed DNA, introduced through methods such as transduction or transfection, may exist physically integrated into the cell's chromosomes, exist temporarily as episomes without replication, or exist as replicable plasmids. If the transformed DNA replicates through the host cell's division, it is considered stably transformed.

Another aspect of the present invention provides a composition for diagnosing cancer comprising the antibody or functional fragment thereof.

Another aspect of the present invention provides a cancer diagnostic kit comprising the composition.

A diagnostic composition comprising the antibody or fragment thereof according to the present invention can be used to diagnose a disease related to the expression or level of ITGA3 or VLA3 or a disease mediated by ITGA3 or VLA3, such as cancer.

The above cancer diagnostic kit may further comprise a composition, solution or device having one or more other components suitable for the analysis method.

Another aspect of the present invention provides a reagent composition comprising the above composition.

**Advantageous effects of disclosure**

Antibodies specific to cancer stem cells or membrane proteins of cancer cells according to one aspect of the present invention exhibit strong affinity for ITGA3 or VLA3 and excellent cell internalization, and thus can be usefully used in research on diagnosis, treatment, or development of therapeutic agents for various cancers in the form of unconjugated antibodies, antibody-drug conjugates (ADCs), and other antibody complexes based on the antibody.

**Brief description of drawings**

Figure 1 shows an overview of the method for identifying cell-internalized antibodies and determining antigens.

Figure 2 shows an overview of the method for discovering antibodies that bind to and internalize cancer stem cell membranes.

Figure 3 is a histogram verifying and selecting antibodies specific to cancer stem cell membranes and cancer cell membrane proteins: Figure 3a is a histogram of verifying and selecting phage-scFv antibodies specific to cancer stem cell membranes and cancer cell membrane proteins; and Figure 3b is a histogram verifying and selecting scFv-Fc antibodies specific to cancer stem cell membranes and cancer cell membrane proteins.

Figure 4a shows representative ITGA3 peptide and ITGB1 peptide sequences identified by LC-MS/MS as VLA-3 antigen of PS4 antibody.

Figure 4b is a graph showing the results of PS4 screening antibodies for target proteins integrin alpha3 and integrin $\beta1$ (left) and the specificity of PS4 antibodies for antigens verified by ELSIA against various recombinant proteins (right).

Figure 4c shows the results of analyzing the specificity of the PS4 antibody against cell membrane target antigens by transfecting HEK293T cells with ITGA3, ITGB1, and ITGA1/ITGB1 cDNA.

Figure 4d shows the results of analyzing the specificity of the antibody by binding the PS4 antibody to the MDA-MB-231 cancer stem cell line after knocking out the ITGA3 gene using CRISPR-Cas9.

Figure 4e shows the results of analyzing PS4 antibody specificity by treating MDA-MB-231 cell lines with ITGA3, ITGB1, and ITGA1/ITGB1 siRNA.

Figure 5a is a graph showing the cell internalization rate of the PS4-002 antibody in the MDA-MB-453 cell line.

Figure 5b shows the results verifying that the antibody co-localizes with lysosomes through immunofluorescence staining.

Figure 6a shows the results of affinity analysis of PS4-002 antibody toward recombinant VLA-3 protein in the presence or absence of cofactor divalent cations ($Mn^{2+}$, $Ca^{2+}$, $Mg^{2+}$) and chelating agent (EDTA).

Figure 6b shows the results of analyzing the affinity between PS4-002 and VLA-3 using a buffer without chelating agent (EDTA).

Figure 6c shows the results of measuring the off-rate of the PS4 antibody using HBS-EP buffer containing 3 mM EDTA. The ligand is PS4-002, and the analyte is VLA-3.

Figure 7a shows the results of analysis of the specificity of the PS4-001 antibody and the expression of the target antigen in various breast cancer, gastric cancer, pancreatic cancer, and colon cancer cell lines.

Figure 7b shows the results of analyzing the specificity of the PS4-002 antibody and the expression of the target antigen in various breast cancer, gastric cancer, pancreatic cancer, colon cancer, and ovarian cancer cell lines.

Figure 7c shows the results of analyzing the specificity of the PS4-004 antibody and the expression of the target antigen in various breast cancer, pancreatic cancer, and colon cancer cell lines.

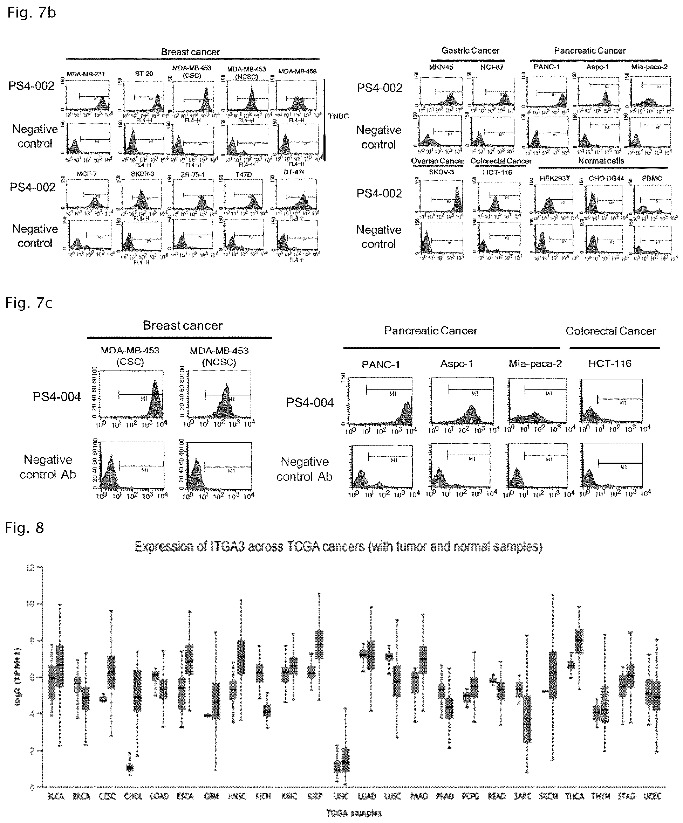

Figure 8 is a graph comparing the expression of ITGA3 in normal tissue and cancer: BLCA, bladder urothelial carcinoma; BRCA, Breast invasive carcinoma; CESC, Cervical squamous cell carcinoma and endocervical adenocarcinoma; CHOL, Cholangiocarcinoma; COAD, Colon adenocarcinoma; ESCA, Esophageal carcinoma; GBM, Glioblastoma multiforme; HNSC, Head and Neck squamous cell carcinoma; KICH, Kidney Chromophobe; KIRC, Kidney renal clear cell carcinoma; KIRP, Kidney renal papillary cell carcinoma; LIHC, Liver hepatocellular carcinoma; LUAD, Lung adenocarcinoma; LUSC, Lung squamous cell carcinoma; PADD, Pancreatic adenocarcinoma; PCPG, Pheochromocytoma and Paraganglioma; PRAD, Prostate adenocarcinoma; READ, Rectum adenocarcinoma; SARC, Sarcoma; SKCM, Skin Cutaneous Melanoma; STAD, Stomach adenocarcinoma; THYM, Thymoma; THCA, Thyroid carcinoma; UCEC, Uterine Corpus Endometrial Carcinoma.

Figure 9a is a graph comparing the expression of ITGA3 in normal tissue and bladder cancer.

Figure 9b is a graph comparing the expression of ITGA3 based on bladder cancer molecular subtypes.

Figure 9c is a graph comparing and analyzing the expression of ITGA3 based on individual bladder cancer stages of each patient.

Figure 10a is a graph comparing the expression of ITGA3 in normal tissue and pancreatic cancer.

Figure 10b is a graph comparing the expression of ITGA3 based on the individual pancreatic cancer stage of each patient.

Figure 11a is a graph comparing the mRNA expression levels of ITGA3 in normal pancreas and pancreatic cancer.

Figure 11b is a graph comparing the overall survival probabilities based on the expression of ITGA3 in pancreatic cancer patients.

Figure 11c is a graph comparing the overall survival probabilities based on the expression of ITGA3 in TNBC patients.

Figure 12 shows a schematic diagram of the production of PS4 antibody variants: WT represents PS4, and the inserted Cys site is indicated by a red circle.

Figure 13a shows the RP-HPLC chromatograms of PS4-002 (red) and PS4-vcMMAE (blue).

Figure 13b shows the MALDI-TOF mass spectra of PS4-002 (a) and PS4-002-vcMMAE (C~E).

Figure 13c shows the enlarged SEC-HPLC chromatogram of PS4-002-vcMMAE.

Figure 13d shows the results of comparative analysis of the efficacy between batches of PS4-002-vcMMAE conjugates.

Figure 14a shows the results of analyzing the target antigen binding ability of antibodies against PS4 and PS4 variant, and the corresponding MMAE drug conjugates, as a function of VLA-3 antigen concentration (left), and the results of analyzing the antigen binding ability of PS4 antibodies and PS4-antibody drug conjugates to the antigen as a function of their concentration (right).

Figure 14b shows the results of analyzing the cytotoxicity of PS4-002 and PS4-002 variants combined with MMAE drug against breast cancer and pancreatic cancer cell lines.

Figure 15 shows the results of analyzing the thermal stability between PS4 antibody and PS4 antibody-drug conjugate.

Figure 16a shows the results of analyzing the *in vitro* efficacy of the PS4 antibody-drug conjugate in various cancer cell lines: pancreatic cancer cell lines Panc-1, Aspc-1; triple-negative breast cancer cell lines BT-20, MDA-MB-468, MDA-MB-231, MDA-MB-453; gastric cancer cell line MKN4; lung cancer cell line A549; and negative control cell line MDA-MB-231 ITGA3 KO.

Figure 16b shows the results of analyzing the *in vitro* efficacy of PS4 antibody and PS4 antibody-drug conjugate in bladder cancer cell lines: bladder cancer cell lines UMUC-3, CoCaB1, SCaBER, SW780, and negative control cell line UMUC3-ITGA3 KO. Control ADC is hlgG-MMAE.

Figure 17a shows the *in vivo* anticancer efficacy results of the PS4-vcMMAE antibody-drug conjugate in a triple-negative breast cancer BALB/c model.

Figure 17b shows the *in vivo* anticancer efficacy results of the PS4-vcMMAE antibody-drug conjugate in a triple-negative breast cancer NOD/SCID model.

Figure 17c shows the *in vivo* anticancer efficacy results of the PS4-vcMMAE antibody-drug conjugate in a triple-negative breast cancer model.

Figure 18 shows the results confirming that the PS4-vcMMAE conjugate inhibits tumor growth in the PANC-1 pancreatic cancer xenograft model.

## Examples

Hereinafter, preferred examples are presented to aid in understanding the present invention. However, the following examples are provided solely to facilitate a better understanding of the present invention, and the scope of the present invention is not limited by the following examples.

## Preparative Example 1. Cell Culture

All cell lines were cultured according to the supplier's instructions unless otherwise specified. Using DMEM and RPMI1640 culture media containing 10% FBS and 1% penicillin/streptomycin, cells were cultured at 37°C in a 5% $CO_2$ atmosphere, respectively. CHO-DG44 cells were cultured in DMEM/F12 HEPES (Invitrogen) containing 10% FBS, 1% penicillin/streptomycin, and HT supplement (sodium hypoxanthine, thymidine mixture) at 37°C in a 5% $CO_2$ incubator. Cancer stem cells (CSCs) were established based on MDA-MB-453, and overexpression of stem cell markers such as CD44/CD133 was confirmed (Kim, E.G et al. Conjugates. Biomolecules 2020, 10, 955.).

## Example 1. Discovery and validation of internalizing antibodies that bind to cancer stem cell lines.

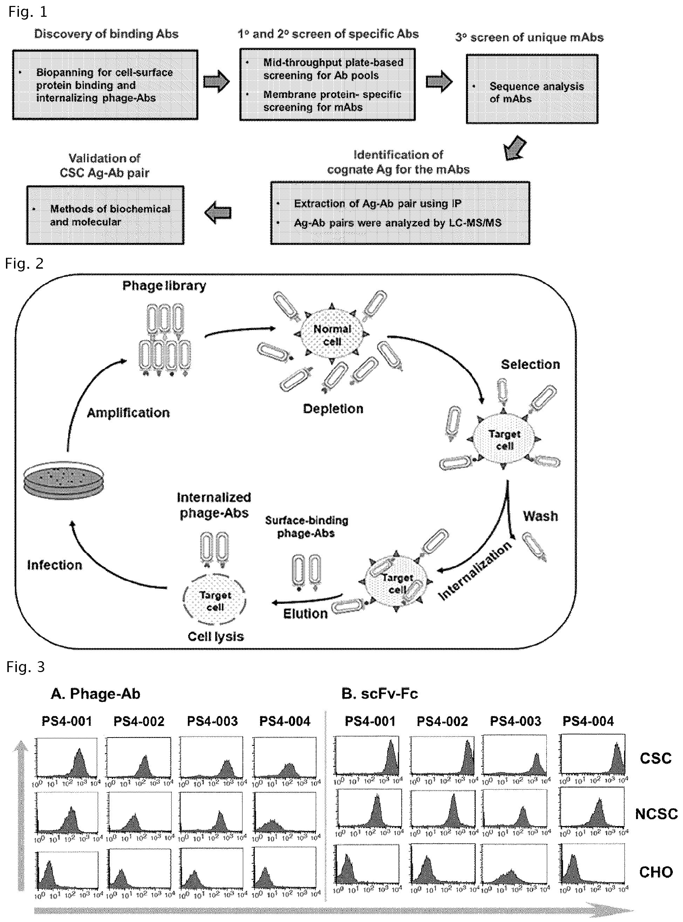

To discover antibodies that recognize the native phenotypic structure and microenvironment of the target protein of cancer stem cell membranes and exhibit excellent intracellular internalization, live-cell phage-display biopanning was performed as outlined in FIG. 1 to obtain antibodies, characterize them, and identify antigens specifically recognized by the antibodies.

Antibodies that bind to and internalize into cancer stem cell membranes were identified by performing the following biopanning technique (Fig. 2). The mixture of $1 \times 10^6$ cancer stem cell lines and $1 \times 10^{10}$ pfu scFv-phage antibodies was incubated at 4°C for 1.5 hours and then centrifuged at 400x g for 5 minutes to separate cells and phage-scFv antibodies. Unbound scFv-phage antibodies were removed by washing five times with DMEM/1% BSA. For the internalization of phage-scFv bound to the cell lines, incubation was performed for 1 hour at 37°C in a $CO_2$ incubator. To isolate antibodies that specifically bind to cancer stem cells, cells were placed in a dibutylphthalate: cyclohexane 9:1 non-miscible organic lower phase and centrifuged at 10,000x g for 10 minutes. After freezing the organic phase using dry ice, the bottom of the tube was cut off to remove the supernatant. The cell pellet was washed with phosphate-buffered saline (PBS), and phage-scFv antibodies bound to the cell membrane were isolated using a solution of 150 mM NaCl, 0.1 M glycine, pH 2.8. The phage-scFv antibodies internalized within cells were recovered by lysing the cells with a 100 mM trimethylamine (TEA) solution.

## Example 2. Selection of specific monoclonal antibodies binding to cancer stem cell lines

Antibodies capable of recognizing the native phenotypic structure/microenvironment of the target protein expressed on the cancer stem cell membrane and possessing internalization ability were selected as follows.

Phage antibodies or maxibody (2.5 μg/mL) were incubated with cells ($1 \times 10^6$) for 1 hour at 4°C to bind, and washed three times with PBS, 1% BSA, 0.03% NaN3, pH 7.4 buffer. Mouse anti-M13 phage antibody (Thermo Scientific) and Alexa Flour® 647 goat anti-mouse antibody (Jackson Immunoresearch) were sequentially added to fluorescently label the phage antibodies specifically bound to the cells. Fluorescence detection of antibody-labeled samples and data analysis were performed using FACS caliber (BD Bioscience) and CellQuest Pro software. To verify the specificity of the selected phage-antibody against the cancer stem cell membrane antigen, the phage-antibody was converted to a scFv-human IgG1 Fc form (referred to herein as Maxibody) in which the Fc domain of human IgG1 is bound, and the fluorescently labeled anti-huIgG antibody was used to verify the same method as above.

Maxibodies were produced similarly to the technique previously reported by our laboratory (Kim, E.G. et al., Biomolecules 2020, 10, 955). Briefly, the scFv portion of the phage-antibody was cloned into the pCEP4 vector containing the human IgG1 Fc domain, and antibody expression was performed in Expi-CHO cells according to the Thermo's manual. The expressed antibodies were purified and separated on a MabselectSuRe column using the ATKA system.

In this manner, the binding of antibodies to cell surface integrin proteins was confirmed in MDA-MB-453 (CSC) and MDA-MB-453 (NCSC) cells. As a result, by comparing and analyzing phage-antibodies that bind to the CHO cell line, the negative control, versus those that bind to cancer stem cells (CSCs), the phage-antibodies that specifically bind to cancer stem cell membrane proteins could be selected (Fig. 3a). In addition, it was verified that the phage-antibody was converted into Maxibody antibodies (scFv-human IgG1 Fc/scFv-Fc) and specifically bound to cancer cell membrane proteins (Fig. 3b). The antibodies selected in this manner were designated PS4-001, PS4-002, PS4-003, and PS4-004, respectively (hereinafter referred to as PS4 antibodies), and their scFv sequences are shown in Tables 1 to 4 below.

**[Table 1]**

| PS4-001 | | amino acid sequence | Sequence number |
|---|---|---|---|
| heavy chain variable region | VH | QVQLVQSGGGLVQPGESLRLSCAASGFSIG**TYWMN**WVRQAPGKGLEWVS**AISGSGGSTYYADSVKG**RFTISRDNSKNTLYLQMNSLRAEDTAVYYCAR**DQGYLPFDY**WGLGTLVTVSS | 25 |
| HCDR1 | **TYWMN** | | 1 |
| HCDR2 | **AISGSGGSTYYADSVKG** | | 5 |
| HCDR3 | **DQGYLPFDY** | | 9 |
| light chain variable region | VL | DIQMTQSPSSLSASVGDRVTITC**RASQDIGNDLG**WYQQKPGKAPKLLIY**GASSLQS**GVPSRFSGRRSATDFTLTIFNLQAEDFASYYC**LQDYSYPLT**FGGGTKVEIKR | 29 |
| LCDR1 | **RASQDIGNDLG** | 13 | |
| LCDR2 | **GASSLQS** | 17 | |
| LCDR3 | **LQDYSYPLT** | 21 | |

**[Table 2]**

| PS4-002 | | amino acid sequence | Sequence number |
|---|---|---|---|
| heavy chain variable region | VH | QVQLVQSGGGLVNPGGSLRLSCAASGFPFS**DHYMS**WIRQAPGKGLEWVS**YISNRGYTIHYADFVKG**RFTISRDNAENTLYLQMNSLRAEDTAVYYCAR**DRGTIDAFDL**WGQGTMVTVSSG | 26 |
| HCDR1 | **DHYMS** | | 2 |
| HCDR2 | **YISNRGYTIHYADFVKG** | | 6 |
| HCDR3 | **DRGTIDAFDL** | | 10 |

(continued)

| PS4-002 | | amino acid sequence | Sequence number |
|---|---|---|---|
| light chain variable re-gion | VL | QSALTQPASVSGSPGQSITIPC**SGTSDDIGRYNYVS**WYQQHPGKAPKLLIY**DVTRRSS**GVSSHFSGSKSGSTASLIISGLQADDEADYFC**SSFTNNRTVV**FGGGTKLTVLG | 30 |
| LCDR1 | **SGTSDDIGRYNYVS** | | 14 |
| LCDR2 | **DVTRRSS** | | 18 |
| LCDR3 | **SSFTNNRTVV** | | 22 |

**[Table 3]**

| PS4-003 | | amino acid sequence | Sequence number |
|---|---|---|---|
| heavy chain variable region | VH | QVQLQESGPRQVKPSETLSLTCVTGGSMS**SYYWS**WIRQSPGKGLEWIG**YIYDTVTDYNPSLKS**RVTISVDTSKNQFSLKLTSVTAVDTAVYYCAR**ETGSWYTFDY**WSPGALVTVSS | 27 |
| HCDR1 | **SSYYWS** | | 3 |
| HCDR2 | **YIYDTVTDYNPSLKS** | | 7 |
| HCDR3 | **ETGSWYTFDY** | | 11 |
| light chain variable region | VL | DIQMTQSPSSLSASVGDRVTITC**RATQDIDNFLN**WYQQKPGKAPKLLIY**EASNLET**GVPSRFSGSGSGTDFTLTISSLQPDDFALYYC**QQSYSSPYS**FGPGTKVDIKR | 31 |
| LCDR1 | **RATQDIDNFLN** | | 15 |
| LCDR2 | **EASNLET** | | 19 |
| LCDR3 | **QQSYSSPYS** | | 23 |

**[Table 4]**

| PS4-004 | | amino acid sequence | Sequence number |
|---|---|---|---|
| heavy chain variable region | VH | QVQLQESGPGLVKPSETLSLTCSVSGGSIS**DYHWS**WIRQFPGKGLEWIG**FISPSAINNYNPSLKS**RVSMSTDTSKNQFSLTLTSVTAVDTAVYYCAR**GGWSLDS**WGQGTLVTVSS | 28 |
| HCDR1 | **DYHWS** | | 4 |
| HCDR2 | **FISPSAINNYNPSLKS** | | 8 |
| HCDR3 | **GGWSLDS** | | 12 |
| light chain variable region | VL | DIQMTQSPSSLSASVGDRVTITC**QASQDITDFLN**WYQQKPGRAPKLLIY**DASNLET**GVPSRFSGSGSGTDFTLTISGLQPDDFALYYC**QQSYTTPYS**FGPGTKVEIKR | 32 |
| LCDR1 | **QASQDITDFLN** | | 16 |

(continued)

| PS4-004 | | amino acid sequence | Sequence number |
|---------|---|---------------------|-----------------|
| LCDR2 | **DASNLET** | | 20 |
| LCDR3 | **QQSYTTPYS** | | 24 |

**Example 3. Analysis and verification of PS4 antibody recognition antigen (target)**

To identify the target antigen of the selective antibody that specifically binds to MDA-MB-453 cancer stem cells, in-situ immunoprecipitation and LC-MS/MS analysis were performed.

Briefly, after incubating the PS4 Maxibody antibody of Example 2 with the target cancer cell line, the antibody and antigen were stably chemically conjugated using BS3 chemical linker, and then the antibody-antigen complex was pulled down. The obtained antibody-antigen complex was separated by SDS-PAGE, and the presence of the complex was verified via Western blot analysis by comparing them with the control group to confirm the location of the target antibody/antigen. Then, in-gel digestion was performed on the anticipated antibody/antigen gel regions identified on the SDS-PAGE. LC-MS/MS analysis was performed on the extracted peptides, and the detected peptide sequences were used to identify the proteins recognized by the antibody using proteome database search tools such as PD 2.3 and PEAKS studio. For confident target protein identification, proteins specifically identified in the sample group containing the PS4 antibody, compared to proteins identified in the negative control group using a label-free quantitative analysis method with proteins identified in the negative control group, were confirmed and selected as final target candidates. Through this process, the PS4 antibody target was identified as ITGA3/ITGB1 (VLA-3).

The identified PS4 target proteins were verified by ELISA and flow cytometry as follows. Recombinant proteins were coated on ELISA plates, blocked with PBS/3% milk, and then incubated with 1 $\mu$g/mL of each selected antibody and control human IgG antibody at room temperature for 1.5 hours. Anti-human Fc-HRP (1/5000 dilution) was added to each plate well and incubated at room temperature for 1 hour. Then, TMB substrate was added and reacted, and the HRP reaction was stopped with 0.25 M sulphuric acid, and the signal was detected at 450 nm.

Flow cytometry was performed as follows. $1 \times 10^6$ HEK293T cells were seeded in 100 mm cell culture plates. On the next day, ITGA3, ITGB1, or ITGA3/ITGB1 expression plasmids were transfected at a DNA:PEI ratio of 1:3 (w:w). After 4 hours, the medium was replaced with DMEM/10% FBS, and the cells were cultured for 48 hours. Binding between the transfected cells and PS4 antibodies was analyzed. ITGA3 and ITGB1 knockout (KO) or knockdown (KD) were performed in MDA-MB-231 cells as follows. For knockdown, MDA-MB-231 cells were seeded in 6-well plates at $1 \times 10^5$ cells/mL and experiments were conducted when the cells reached 60-80 confluence. ITGA3 and ITGB1 siRNA were mixed with siRNA transfection medium at 10$\mu$M each and applied to the cell line, which was then cultured for 6 hours. After 6 hours, the medium was replaced with DMEM/10% FBS/1% P/S, and the cells were cultured for 48 hours. Transfected cells were cultured and analyzed for binding to the PS4 antibody.

MDA-MB-231 cells with ITGA3 gene knockout were generated as follows. sgRNAs targeting exon 17 (CCA GACCTCGCTTAGCATGTGGG) of the ITGA3 gene were subcloned using the CRISPR-Cas9-2A-Puro V2.0 (Addgene) system. MDA-MB-231 cells were transfected with CRISPR-Cas9/sgRNA and cultured at 37°C, 5% $CO_2$ for 72 hours. Afterwards, puromycin, the selection marker for the CRISPR-Cas9 vector, was added at a concentration of 250 ng/mL and selected for 18 days. Then, the target protein knockout efficiency was measured using flow cytometry (FACS calibur, FACS Aria, BD Biosciences), and knockout cells were isolated. MDA-MB-231 cells with ITGA3 knockout, i.e., MDA-MB-231/ITGA3 KO cell lines, were analyzed by flow cytometry using 3 $\mu$g/mL PS4 antibody and anti-human Fc-gamma specific Alexa Fluor-647 (1:800 dilution, Jackson ImmunoResearch Laboratories Inc.). In addition, MDA-MB-231/ITGA3 KO cell lysate (30 $\mu$g) was separated by SDS-PAGE and analyzed by Western blot using anti-ITGA3 (Antibodies-online) and anti-mouse Fc-gamma specific-HRP. Sample loading was compared using anti-beta-actin.

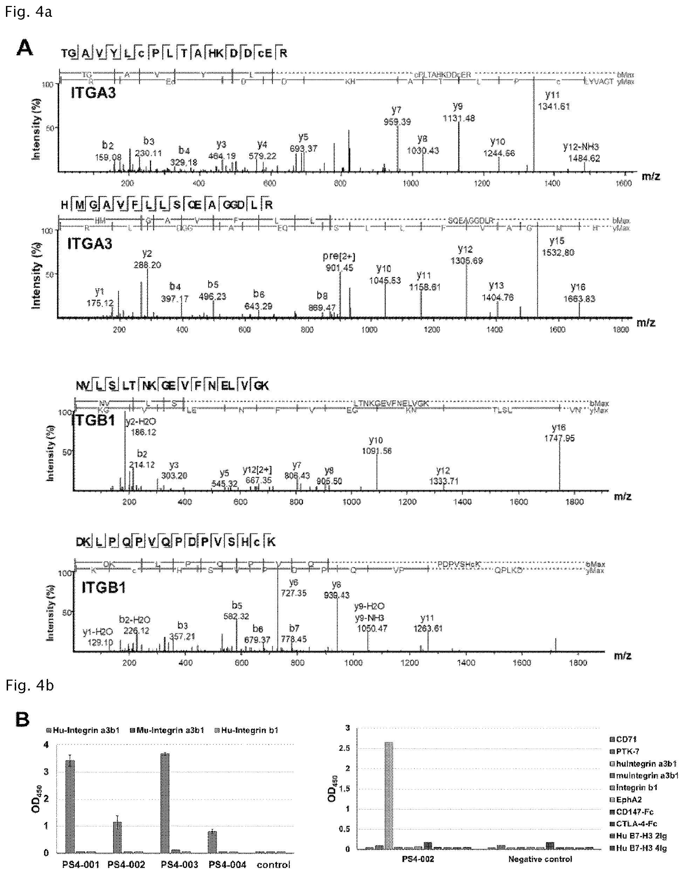

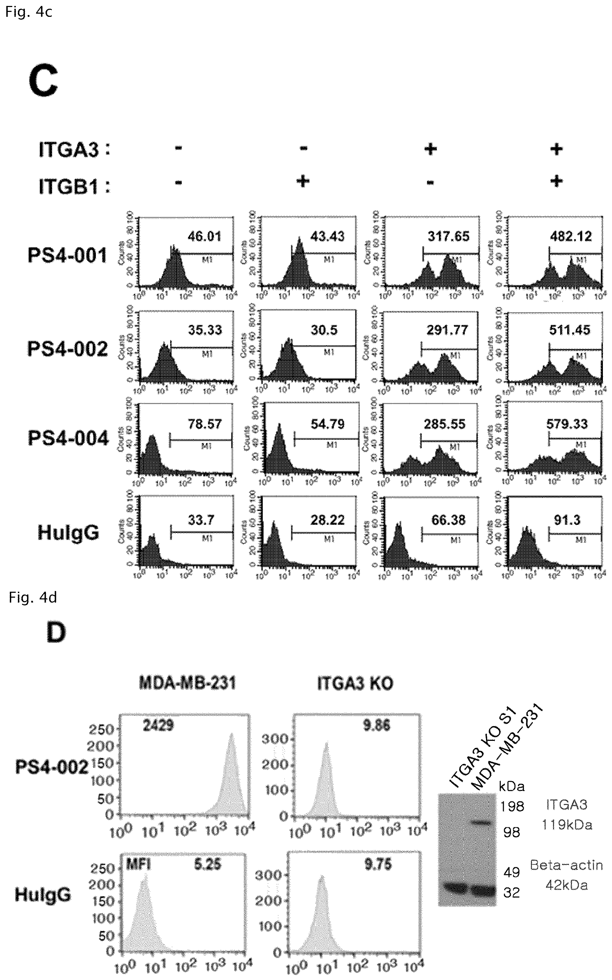

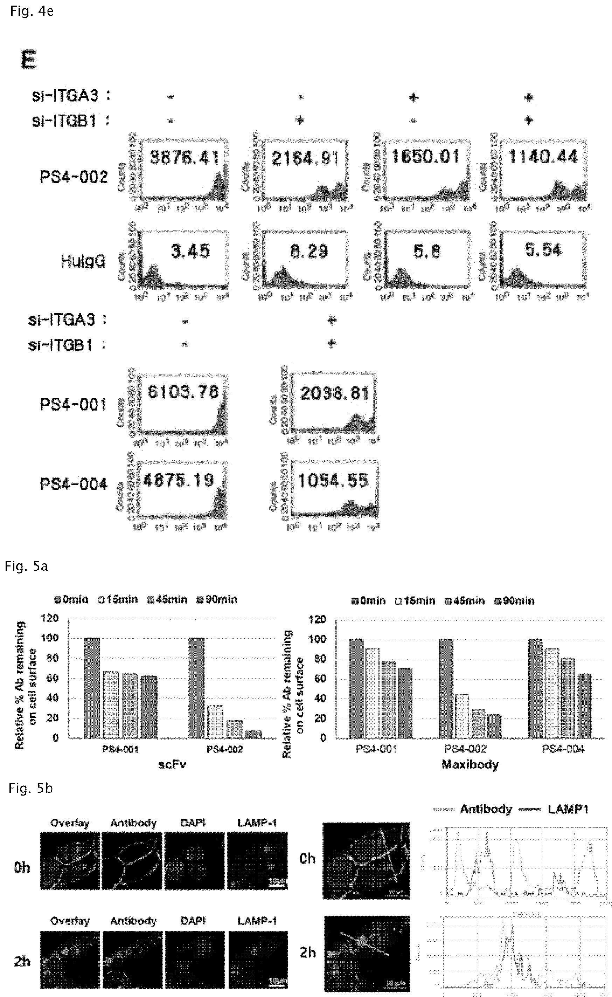

The PS4-002 cancer stem cell antibody thus selected, and the pulled-down cancer stem cell membrane target antigen were analyzed by LC-MS/MS. Compared to the negative control group, the cancer stem cell membrane target antigen recognized by the PS4 antibody was identified through label-free proteome quantification, analysis based on electrophoretic mobility of protein mass on SDS-PAGE using cross-linked techniques, and peptide sequence identification in the target samples. The integrin ITGA3 and ITGB1 peptides as the target proteins of the PS4 antibody specific to MDA-MB453 were identified by using a label-free quantitative proteomics analysis method based on spectral counting (Fig. 4a). Based on the previous report that integrin ITGA3 forms a specific heterodimer with integrin ITGB1, the target protein of the PS4 antibody was presumed to be ITGA3/ITGB1, i.e., VLA-3. In addition, the identified monoclonal PS4 antibody was confirmed by ELISA to specifically bind to recombinant human VLA-3 protein, particularly to the ITGA3 subunit (Fig. 4b). Furthermore, to analyze whether the PS4 antibody recognizes and specifically binds to the cell membrane VLA-3, HEK293T cells with low VLA-3 expression were transiently overexpressed with ITGA3, ITGB1, or ITGA3/ITGB1. In the experimental group, compared to the control group, the selected PS4 antibody exhibited increased binding affinity to HEK293T/ITGA3 and even stronger binding to HEK293/ITGA3/ITGB1 (i.e., VLA-3) (Fig. 4c). In addition, it was confirmed that the binding of the PS4 antibody was inhibited or reduced upon ITGA3 knockout or ITGA3/ITGB1 knockdown in MDA-MB-231 cells overexpressing VLA-3 (Figs. 4d and 4e). Knockout of ITGA3 in MDA-MB-231 confirmed the absence of ITGA3 protein expression by Western blot, and FACS analysis verified that the PS4 antibody did not bind to MDA-MB-231/ITGA3 KO cells (Fig. 4d).

The PS4 antibody showed increased binding affinity specifically to HEK293T cells overexpressing ITGA3, while in MDA-MB-231 cells, binding affinity tended to decrease in both ITGA3 and ITGB1 knock-out/down samples. Therefore, the PS4 antibody exhibited relatively higher binding affinity to ITGA3 than to IGTB1, but it also specifically binds to the ITGA3/BETA1 complex.

## Example 4. Analysis of cell internalization characteristics of PS4 antibody

The cell internalization characteristics of the PS4 antibody according to one specific example were analyzed by flow cytometry and immunofluorescence as follows.

Flow cytometry was performed similarly to Example 3. After binding cells to scFv or Maxibody PS4 antibodies, incubation was performed at 37°C for various periods of time to compare and analyze PS4 antibodies bound to the cell membrane surface due to antibody internalization.

The cell internalization characteristics were observed using immunofluorescence as follows. CSCs cultured at approximately 70% confluence in confocal imaging dishes were incubated with scFv or Maxibody PS4 antibodies for 1 hour on ice to facilitate binding. After washing once with cold PBS, the samples were placed in culture medium and incubated for 2 hours at 37°C in a $CO_2$ incubator, and the control samples were placed on ice. After the cell internalization reaction was completed, all samples were washed twice with cold PBS and then fixed with 4% paraformaldehyde at room temperature for 20 minutes. The cells were then permeabilized with 0.1% Triton X-100 for 5 minutes and washed twice with PBS. ScFv-Fc was stained with Alexa594-labeled anti-human IgG (Jackson Immunoresearch) as the secondary antibody, and lysosomes were stained with LAMP1 antibody (BioLegend) and Alexa Fluor® 647-labeled F(ab')2 anti-mouse IgG. Nuclei were stained with 300 nM DAPI. After all reactions were completed, cells were covered with mounting medium and a coverslip, and then images were analyzed using a confocal microscope (ZEISS LSM 880).

As a result, flow cytometry analysis confirmed that PS4 antibodies in both scFv and scFv-Fc forms exhibited rapid cellular internalization into cancer stem cell lines (Fig. 5a). In particular, the PS4-002 PS4 antibody demonstrated ~70% internalization of antibodies when incubated at 37°C for 15 minutes. In addition, immunofluorescence analysis confirmed that the PS4 antibody possessed the ability to internalize into cells over time (Fig. 5b). Furthermore, it was confirmed that the PS4 antibody was internalized into cells and then co-localized with the lysozyme marker LAMP1.

As described above, the rapid cellular internalization ability of the PS4 antibody was verified by flow cytometry and immunofluorescence. These results indicate that the PS4 antibody is suitable for use as an antibody in drug delivery systems such as antibody-drug conjugate platforms.

## Example 5. Analysis of target binding affinity of PS4 antibody

The binding affinity between the PS4 antibody according to one embodiment and the target protein (Acrobiosystem) was confirmed through surface plasmon resonance (SPR) analysis as follows.

Analysis was performed using the Octet Red instrument (Sartorius) according to the manufacturer's instructions. First, 50 nM of PS4-002 antibody was loaded onto AHC sensor (Anti-hIgG Fc, Sartorius) for 20 minutes. After washing with kinetic buffer (1 mM phosphate, 15 mM NaCl, 0.002% Tween 20, 0.005% Azide, 0.1 mg/mL BSA, pH 7.4) for 10 minutes, the VLA-3 recombinant protein (50 nM) and analyte containing 2 mM $Ca^{2+}$, $Mg^{2+}$, $Mn^{2+}$, or EDTA were added to the kinetic buffer to measure the association of the VLA-3 antigen with the PS4 antibody for 1 hour. Then, the sample was washed with kinetic buffer, and the disassociation was measured for 2 hours. The binding affinity of the PS4 antibody was measured in the same manner as above with some modifications as follows. AHC biosensors hydrated in kinetic buffer for 30 min were loaded with 100 nM PS4 antibody for 10 min. After washing the biosensors with kinetic buffer, association with 25-120 nM target recombinant protein was performed for 40 min, followed by dissociation for 50 min. Affinity data measured with Octet Red were analyzed using Fortebio Data Analysis 8.2 software using a 1:1 fitting model.

Additionally, the binding affinity of PS4-002 antibody was analyzed using Biacore T200 instrument. According to the BIAcore's SPR method, the effect of kinetic running buffer HBS-EP and HBS-EP+ running buffer containing 3 mM EDTA on the binding affinity of PS4 antibody was investigated with following modified approach. After immobilizing PS4 antibody on Sensor Chip CM5, target recombinant proteins were added at concentrations of 250, 125, 62.5, 31.3, 15.6, 7.8, and 3.9 nM, and the KD values were measured using the running buffer (10 mM HEPES, 150 mM NaCl, 3 mM EDTA, and 0.05% v/v Surfactant P20).

Integrin proteins are classified into three types depending on their activation level: Bent closed, Extended closed, and Extended open. It has been reported that the binding affinity between ligand and integrin protein varies greatly depending on the structure of the integrin (Peterson RJ and Koval M (2021) Above the Matrix: Functional Roles for Apically Localized Integrins. Front. Cell Dev. Biol. 9:699407). $Ca^{2+}$ and EDTA are known to control the binding of VLA-3 to ligand by contributing to the conversion of ITGA3/BETA1 protein to the inactive form, whereas $Mg^{2+}$ and $Mn^{2+}$ contribute to the conversion of ITGA3/BETA1 protein to the active form. Therefore, in this experiment, the difference in binding strength of the PS4 antibody to the active and inactive forms of ITGA3/BETA1 protein was confirmed, and the antibody binding affinity based on the divalent cation affecting integrin structure was analyzed.

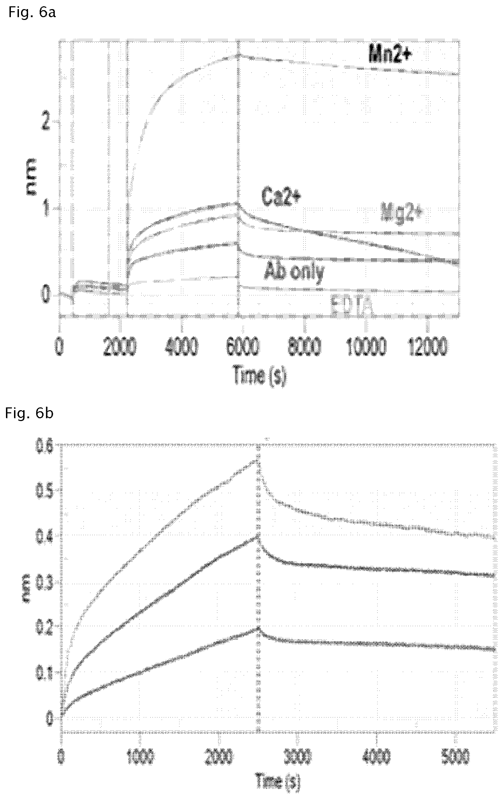

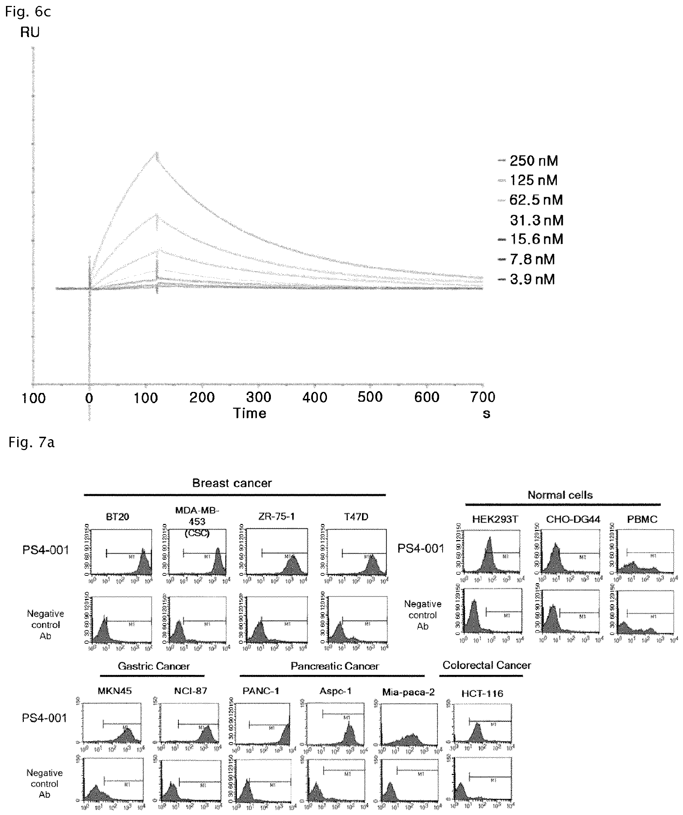

As a result, in the comparison group containing $Ca^{2+}$ or EDTA, which are known to inhibit the binding of VLA-3 to ligands, the binding affinity between VLA-3 and antibodies was reduced, and a particularly rapid dissociation rate (off-rate $10^{-3} \sim 10^{-4}$) was observed (Fig. 6a, Table 5). On the other hand, in the comparison group containing $Mn^{2+}$ and $Mg^{2+}$, which are known ITGA3/BETA1 activators, it was confirmed that the VLA-3 antigen-PS4 antibody binding affinity (off-rate $10^{-5} \sim 10^{-6}$) increased. In the absence of divalent cations, the KD between VLA-3 and PS4 antibodies was measured to be 2.15 + 1.2 nM. In addition, the HBS-EP running buffer commonly used in the BIAcore technique for measuring affinity contains 3 mM EDTA. Therefore, it can be seen that the KD values measured on the Biacore using HBS-EP buffer are higher compared to those measured pm the Octet Red instrument. The reason for this is believed to be that the measured KD values between Biacore and Octet Red are affected by the off rate as described above.

[Table 5]

| Additive | $K_D$ (nM) | $k_{on}$ (1/Ms) | $k_{off}$ (1/s) | R^2 | Memo |
|---|---|---|---|---|---|
| None | 216 | $2.09 \times 10^4$ | $4.5 \times 10^{-3}$ | 1.02 | 3 mM EDTA in the running buffer |
| None | $2.15 \pm 1.2$ | $3.25 \times 10^4$ | $5.90 \times 10^{-5}$ | 0.991 | 0 mM EDTAin the running buffer |
| 2mM $Ca^{2+}$ | 6.46 | $3.24 \times 10^4$ | $2.09 \times 10^{-4}$ | 0.965 | - |
| 2mM $Mg^{2+}$ | 0.85 | $4.46 \times 10^4$ | $3.78 \times 10^{-5}$ | 0.794 | - |
| 2mM $Mn^{2+}$ | 0.23 | $3.65 \times 10^4$ | $8.46 \times 10^{-6}$ | 0.940 | - |
| 2mM EDTA | 2,170 | $1.83 \times 10^3$ | $3.97 \times 10^{-3}$ | 0.942 | - |

As such, it was confirmed that divalent cations significantly influence the binding affinity between the ligand and ITGA3/BETA1, as well as the heterodimer complex formation of ITGA3/BETA1. In this experiment, it was confirmed that the affinity between PS4 antibody and ITGA3/BETA1 protein was high in the activator form, and it was inferred that the epitope of the antibody is related to the binding part of the ligand and ITGA3/BETA1 protein. In particular, it is presumed that dynamic structural changes in ITGA3/BETA1 are caused according to cancer progression due to changes in the concentration of divalent cations in the microtumor environment, and it is suggested that the antibody according to one specific example is a cancer-specific antibody.

**Example 6. Analysis of binding characteristics of cancer stem cell antibodies**

The binding affinity of the PS4 antibody according to one specific example to various cancer types was confirmed using flow cytometry.

First, PS4 antibodies bound to cell membrane antigens were labeled using selected PS4 maxibody antibodies (0.5 μg/mL) and anti-human Alexa Flour® 647 secondary antibody (Jackson Immunoresearch). Fluorescence detection and data analysis of the labeled antibodies were performed using FACS Calibur (BD Bioscience) and CellQuest Pro software. Human IgG was used as the control antibody.

In addition, the differences in expression and prognosis of the target protein of the PS4 antibody in normal and cancer tissues were analyzed using various database tools as follows. Using a comprehensive web portal (ualcan.path.uab.edu/index.html) for performing in-depth analysis of TCGA gene expression data, integrin RNA expression levels in normal and cancer cells were determined. UALCAN can estimate the impact of gene expression levels and clinicopathological features on patient survival using RNA-seq and clinical data from 31 cancer types in the TCGA database. In this experiment, using UALCAN's "TCGA Analysis" module and datasets for 24 cancer types, ITGA3 expression data in normal and cancer cells were obtained and analyzed. Kaplan-Meier Plotter is a meta-analysis-based platform (kmplot.com/analysis/index.php?p=background) that can discover and validate survival biomarkers, analyze genes (mRNA, miRNA, proteins) related to breast survival, and analyze prognosis. Expression of ITGA3 was determined by median (or upper/lower quartile). Patient groups for analysis were defined based on overall survival, and ITGA3 expression and patient prognosis were analyzed. For breast cancer, HER2-, ER-, and PR-negative groups were separately selected to analyze ITGA3 expression and patient prognosis in triple-negative breast cancer. The 95% hazard ratio (HR) confidence interval and log-rank P value were also calculated for each cancer.

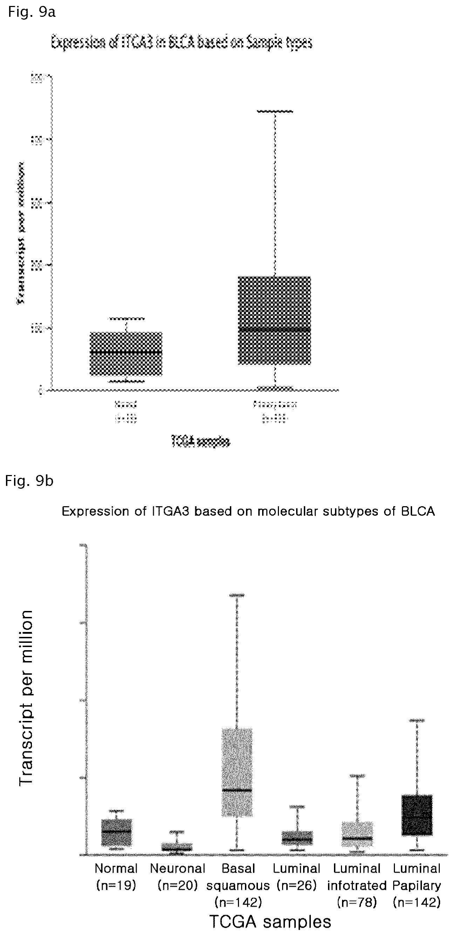

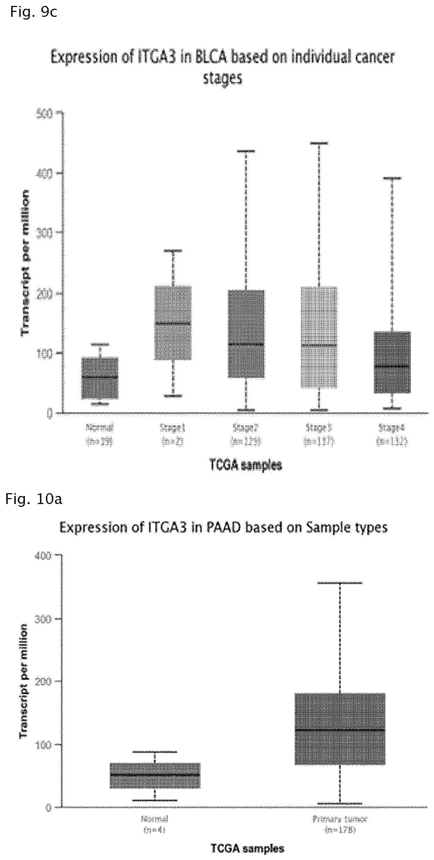

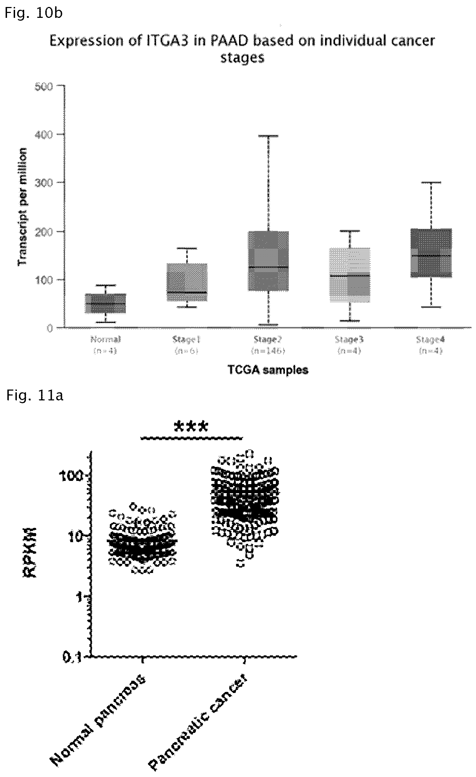

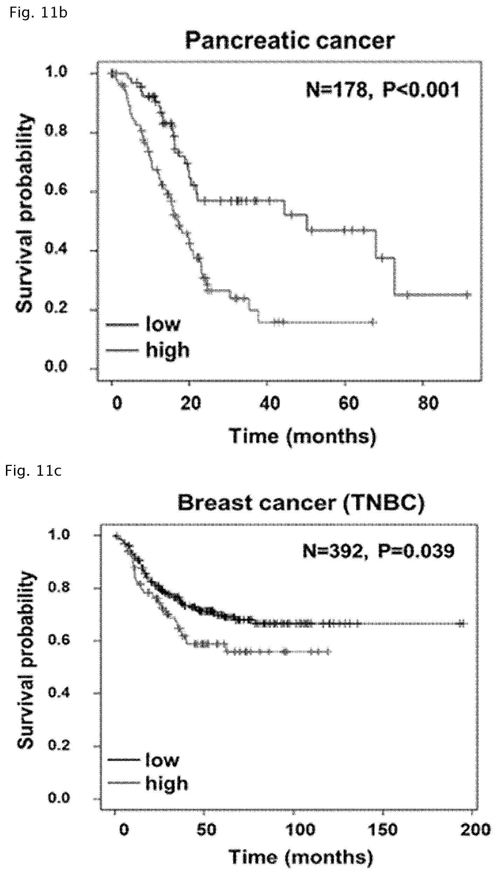

As a result, it was confirmed that the PS4 antibody according to one specific example binds to breast cancer, including triple-negative breast cancer (TNBC), gastric cancer, pancreatic cancer, ovarian cancer, colon cancer, and pediatric brain cancer cell lines (Figs. 7a to 7c). On the other hand, it showed relatively weak binding affinity to normal cell lines such as HEK293T, CHO-DG44, and PBMC cell lines, verifying that the antibody according to one specific example specifically binds to a cancer cell membrane target protein. Furthermore, the expression levels of VLA-3 in various cancers were analyzed by immunohistochemical (IHC) of genes and patient tissues to confirm that the antigen recognized by the PS4 antibody, i.e., VLA-3, is overexpressed in various cancers and is associated with cancer (Figs. 8 to 11). In particular, it was confirmed that ITGA3 mRNA was overexpressed in cancer compared to normal pancreas in pancreatic cancer, and that the higher the expression, the worse the patient prognosis. In breast cancer, ITGA3 expression yields positive outcomes for patient prognosis, but in triple-negative breast cancer, it was confirmed that the higher the expression of ITGA3 mRNA, the worse the patient prognosis.

These results indicate that the PS4 antibody according to one embodiment can be used as a diagnostic and therapeutic agent for various solid tumors with clinical unmet needs, such as breast cancer, including triple-negative breast cancer (TNBC), gastric cancer, pancreatic cancer, colorectal cancer, melanoma, head and neck cancer, and bladder cancer. Furthermore, since VLA-3 is predicted to have a greater effect on cancer cell migration and metastasis than on cell growth/proliferation, it can also be used as a combination therapy with existing cancer treatments.

## Example 7. Production of PS4 Maxibody and Maxibody variants

To produce antibody-drug conjugates (ADCs) with adjusted drug-to-antibody ratios (DARs), five types of maxibody antibody variants, including PS4 wild type (WT), were produced as follows.

PS4 maxibody variant 1 (huFc-K-C variant) was synthesized by substituting the terminal Lysine 478 of huFc portion of PS4-002 maxibody with Cysteine (K478C). PS4 maxibody variant 2 (Linker-C variant) was synthesized by inserting Cysteine into the scFv portion linker of the PS4 antibody to synthesize (GGGGSGGGGSCGGGGS) Linker-C (SEQ ID NO: 47). PS4-maxibody variant 3 (Linker-C/huFc-K-C) was synthesized by fusing the huFc-K-C variant with the Linker-C variant. In addition, variant 4 WT-DeltaC (WT-ΔC), for producing a low DAR antibody-drug conjugate, was produced by preparing primers 5'-GTAAGCTTGAGCCCAAATCTGACAAAACTTATACATGCCC-3' and 5'-GGGCATGTATAAGTTTT GTCAGATTTGGGCTCAAGCTTAC-3' according to the mutagenesis kit (Agilent) manual and removing Cys233. PS4 maxibody variants were expressed, purified, and analyzed in the same manner as the PS4 maxibody described above.

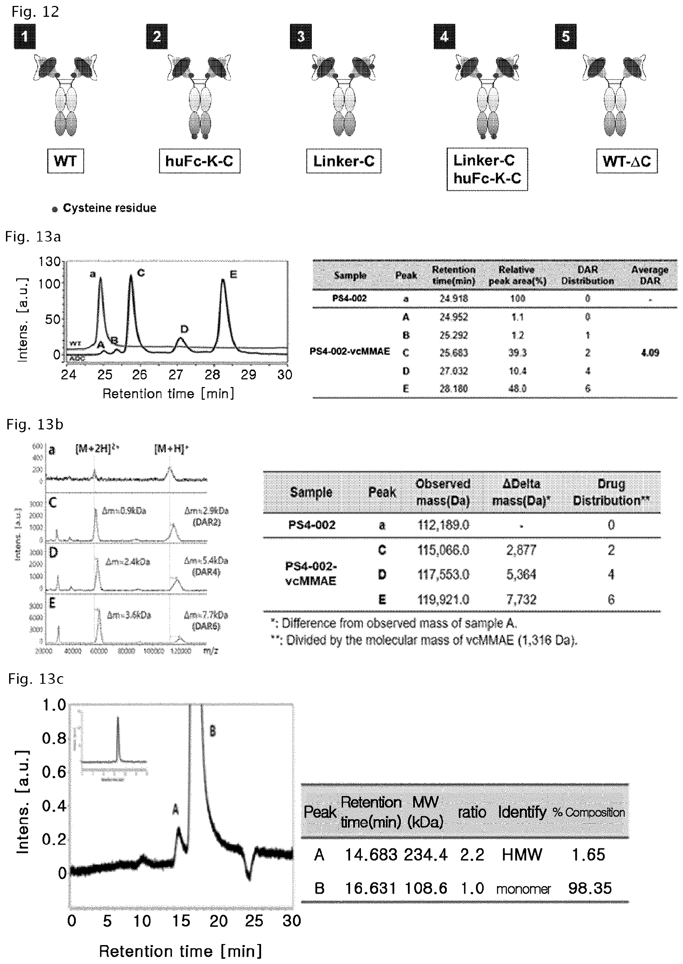

The DAR of an antibody-drug conjugate (ADC) is a critical factor in ACD efficacy. In other words, DAR adjustment based on the potency of the payload used in the ADC manufacturing is crucial. Furthermore, the antibody binding site where the payload is conjugated, i.e., the payload binding site, also influences the overall efficacy of the antibody. Therefore, as described above, to adjust the payload binding amount (antibody:payload ratio) and the binding site (payload conjugation site) on the PS4 maxibody antibody, three PS4 maxibody variants were prepared by inserting a cysteine amino acid for payload conjugation (Figure 12): a PS4 maxibody huFc terminal 447C mutation (huFc-K-C); a Cys addition to the VH -VL linker (Linker-C); and a Linker-C/huFc-K-C variant, which is a combination of the huFc-K-C and Linker-C variants, was confirmed by DNA sequencing.

By using cysteine in the production of antibody-drug conjugates, additional drugs can be conjugated to the inserted cysteine residues, enabling the production of antibody-drug conjugates with diverse DAR properties at various positions. Furthermore, maxibody variants can be applied to the optimization of maxibody-based drug conjugates (MDCs).

## Example 8. Preparation and analysis of PS4 antibody-drug conjugate (ADC)

The cytotoxic drug monomethyl auristatin E (MMAE) was conjugated to PS4 WT and three PS4 variants using the method described in the paper to produce PS4 maxibody-vcMMAE complexes (PS4-vcMMAE MDC) (Mol Cancer Ther (2008) 7 (8): 2486-2497). Briefly, the antibody was partially reduced with 2.5-fold molar excess Tris(2-carboxyethyl) phosphine (TCEP) at 37°C for 1.5 h. The reduced antibody was conjugated with vcMMAE (Medchemexpress) on ice for 0.5 hours, and then the conjugation reaction was quenched by adding 5.3-fold N -acetylcysteine. After the reaction was completed, excess vcMMAE, TCEP, etc. were removed from the mixture using a 10 kDa Amicon concentrator, and the buffer composition was changed to 10 mM Imidazole 2.5% glycerol in DPBS pH 6.4.

The drug-to-antibody ratio (DAR) of the produced PS4-vcMMAE ADC was analyzed by RP-HPLC-MALDI-TOF MS. The DAR of PS4-vcMMAE ADC was analyzed using a Dionex Ultimate 3000-RSLC system with a polymeric reversed-phase column (PLRP-S, Agilent Technologies). The gradient elution was performed at 70 °C with 10-95% mobile phase B (ACN, 0.05% TFA), and mobile phase A was 0.05% TFA in water. The gradient started at 10% mobile phase B (ACN, 0.05% TFA) for 10 min at a flow rate of 0.25 mL/min, then linearly increased to 80% mobile phase B over 30 min, and then to 95% mobile phase B. All DAR fractions were manually collected during sample injection and concentrated using a 10K Ultra Centrifugal filter (Millipore, Billerica, MA, USA). The intact mass of the DAR fraction was measured using matrix-assisted laser desorption/ionization time-of-flight mass spectrometry (MALDI-TOF, Bruker Daltonics). The concentrated DAR fraction (1 μL) was air-dried on a polished stainless steel 96-well MALDI plate. Subsequently, 1 μL matrix solution (1:1 v/v) containing 20 μg/μL sinapinic acid in 30% ACN/0.1% TFA was overlaid, air-dried, and analyzed by MALDI-TOF MS. To optimize experimental conditions, the high molecular weight range (20-160 KDa) was manually calibrated using a mixture of BSA and huIgG. A 20x detector gain with a sampling rate of 0.5 GS/s was used. The spectra were obtained based on an average of 1,600 shots at different positions at a frequency of 60 Hz from the same sample point. The weight average differential aperture ratio (DAR) was measured as follows:

$$\text{Weight average DAR} = \Sigma \left\{ (\text{Drug load} \times \text{Relative peak area (\%)}) / 100 \right\}$$

Monomer and aggregate of PS4 ADC were analyzed using a size exclusion column (SEC, TSKgel G3000SWXL, Tosoh Bioscience). Isocratic elution was performed at 0.5 mL/min for 30 min at 25°C using 0.1 M phosphate buffer and 0.1 M sodium sulfate, pH 6.7. To improve the accuracy of molecular weight estimation, the system was calibrated using Gel Filtration Standards (MW 1,350-670,000, Bio-Rad) according to the manufacturer's instructions. The equilibrium distribution coefficient (Kav) was measured as follows:

$$Kav = (Ve - Vo) / (Vc - Vo)$$

\* Vc : Total column volume(geometric), Ve: Elution volume, Vo : Void volume

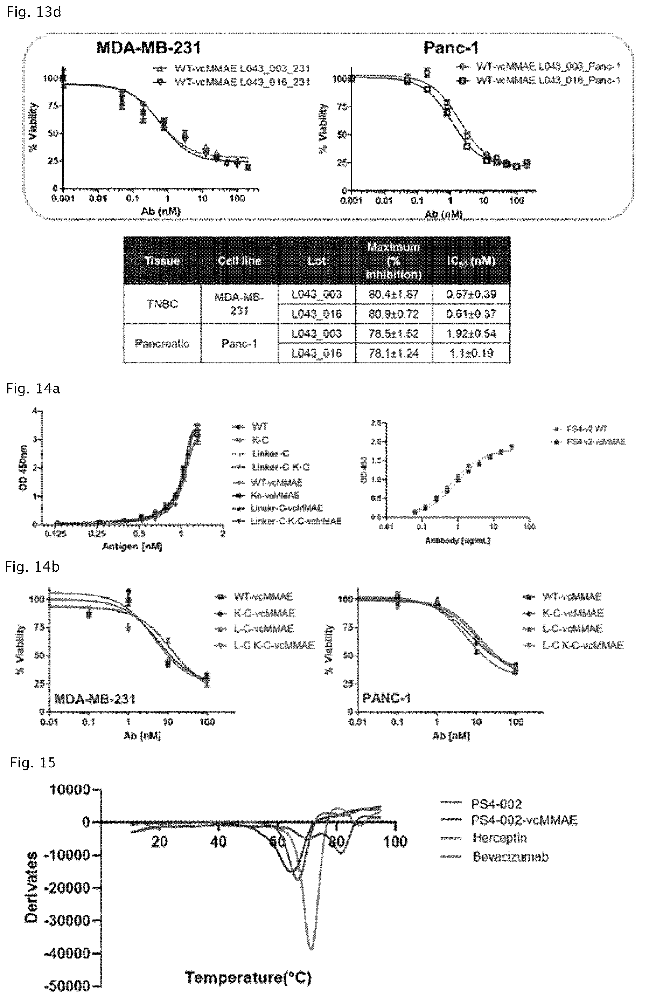

As a result, RP-HPLC analysis confirmed that the retention time increased based on hydrophobicity due to the increased payload distribution of PS4-002-vcMMAE, unlike PS4-002 (Fig. 13a). Each RP-HPLC peak fraction was collected, and the molecular weight was measured by MALDI-TOF-MS to determine the DAR (Fig. 13b). The average DAR of PS4-002-vcMMAE was converted to 4.09 using the weight average DAR formula. The DAR for PS4 variant-vcMMAE was measured and converted using the same method. It was confirmed that the MDC of PS4 and PS4 variants 1 to 3 bound to vcMMAE all had a DAR of 3.6 to 4.5, which is approximately ~4 DAR. In contrast, PS4 variant 4 was confirmed to bind with ~2 DAR. Therefore, we established an MDC platform capable of producing PS4 MDC with DAR 2 or DAR 4 depending on the efficacy of cytotoxic drugs and analyzed the characteristics and efficacy of MDC based on this. In addition, when the monomer and aggregates of PS4-vcMMAE were analyzed by SEC-HPLC, PS4-vcMMAE yielded >95% monomers (Fig. 13c, Table 6). On the other hand, while optimization of PS4 variant-drug conjugate formation is required, it was confirmed that the MMAE drug conjugate of the PS4 variant could also achieve >90% monomer yield (Table 6). Furthermore, as a result of analyzing the quality of PS4-vcMMAE conjugates by DAR, % monomer, and in vitro efficacy assays, it was confirmed that there was no significant variation from lot to lot in the production of PS4-vcMMAE conjugates (Table 6). In addition, the efficacy of PS4-vcMMAE produced in different batches was analyzed on MDA-MB-231 and PANC-1 cell lines, and it was confirmed that the cell growth inhibition efficacy was very similar regardless of the production batch of PS4-vcMMAE (Fig. 13d).

**[Table 6]**

| PS4 format | PS4 MDC | DAR (HPLC) | % monomer |
|---|---|---|---|
| WT | WT-vcMMAE | 4.09 | 98.35 |
| Mutant 1 | K-C-vcMMAE | 3.60 | 91.1 |
| Mutant 2 | L-C-vcMMAE | 4.52 | 92.3 |
| Mutant 3 | L-C-K-C-vcMMAE | 3.92 | 93.8 |
| Mutant 4 | WT-ΔC-vcMMAE | 1.5 | 83.4 |

**Example 9. Analysis of binding characteristics between PS4 antibody-drug conjugate and antigen**

To analyze the antigen binding affinity of PS4 antibodies and PS4 antibody-drug conjugates according to one specific example, ELISA was performed on the target antigen.

First, VLA-3 (0.125-1.5 nM, R&D system) was coated, and then 1 μg/mL of each antibody and antibody-drug conjugate variant was added to bind to the antigen. Non-specifically bound antibodies were removed by washing with PBS/0.05% Tween-20 buffer, and then anti-human-Fc HRP antibody and TMB substrate were added and reacted. Antigen-antibody binding was analyzed by measuring the absorbance A450 nm. Each sample was tested three times.

As a result, all PS4 WT and variant antibodies exhibited EC50 value of ~1 nM with the VLA-3 antigen (Fig. 14a). In addition, it was verified that there was no difference in the binding affinity to the target antigen between each produced PS4 WT and its variants and the corresponding antibody-drug conjugate. In addition, it was confirmed that PS4 and PS4 variants conjugated with MMAE demonstrated similar cytotoxic efficacy against breast cancer cell line (MDA-MB-231) and pancreatic cancer cell line (PANC-1) (Fig. 14b). Accordingly, it was confirmed that the PS4 antibodies with various binding sites according to one embodiment and the binding method using the same did not affect the binding between the antigen and the antibody. It was also verified that the drug can be conjugated to the optimal antibody site according to the cytotoxic drug characteristics.

### Example 10. Analysis of thermal stability characteristics of PS4 antibody-drug conjugate

Real-time thermal stability experiments were performed to analyze the thermal stability of PS4 antibodies and antibody-drug conjugates according to one specific example.

The method and principle of the experiment were as follows. As the temperature increases, the folded protein denatures, exposing hydrophobic regions, which bind to the SYPRO Orange detection dye, resulting in fluorescence. The point at 1/2 RFUmax is designated as the melting point (Tm). 22.5 $\mu$L of the assay sample and 2.5 $\mu$L of 50x SYPRO stock solution were added to a 96-well PCR plate (VOLO Semi-Skirted PCR Plate) so that the final concentration of the sample used in the experiment was 2 $\mu$M, and the fluorescence was measured using Quantstudio Real-Time PCR (Thermo Fisher scientific) while increasing the temperature by 0.5°C per 10 s from 10.0°C to 95°C. Each sample was tested three times. The fluorescence value of the antibody buffer was set as the background and subtracted, and the value with the greatest slope in the aesthetic of the fluorescence value (dTRFU) was analyzed as the Tm of the corresponding protein.

As a result, the Tm values of PS4 maxibody and PS4-vcMMAE complex were measured to be almost the same, $66.67 \pm 0.24$°C and $64.67 \pm 0.24$°C (Fig. 15). These results indicate that thermal instability is not induced by binding drugs to PS4 antibodies. In addition, it was confirmed that PS4 maxibody and PS4 maxibody-drug conjugate (MDC) maintain Tm values in a range similar to that of FDA-approved anti-VEGF (bevacizumab, Avastin) antibodies (Tm 69.94°C). This means that maxibody-based PS4 and PS4-drug conjugate according to one specific example exhibit thermal stability comparable to that of full-IgG antibodies such as Avastin.

### Example 11. In vitro efficacy analysis of PS4 antibody-drug conjugate

The *in vitro* efficacy of the PS4 antibody-drug conjugate according to one specific example was confirmed as follows.

First, an appropriate number of cells ($3 \times 10^3$ to $1 \times 10^4$ cells) for each cancer cell line were dispensed into each well of a 96-well plate and cultured for 24 hours at 37°C, 5% $CO_2$, and then the medium was replaced with fresh growth medium. Then, sequentially diluted antibody-drug conjugates were added to each well, and the cells were cultured for 4 days under 37°C, 5% $CO_2$ conditions, and then treated with AlamarBlue (Invitrogen). The fluorescence values were measured at Ex530 and Em590 every 2, 4, and 6 hours (PerkinElmer Victor x4). The obtained fluorescence values were analyzed with Prism4 (GraphPad) to determine the $EC_{50}$ value. Each experiment was repeated three times.

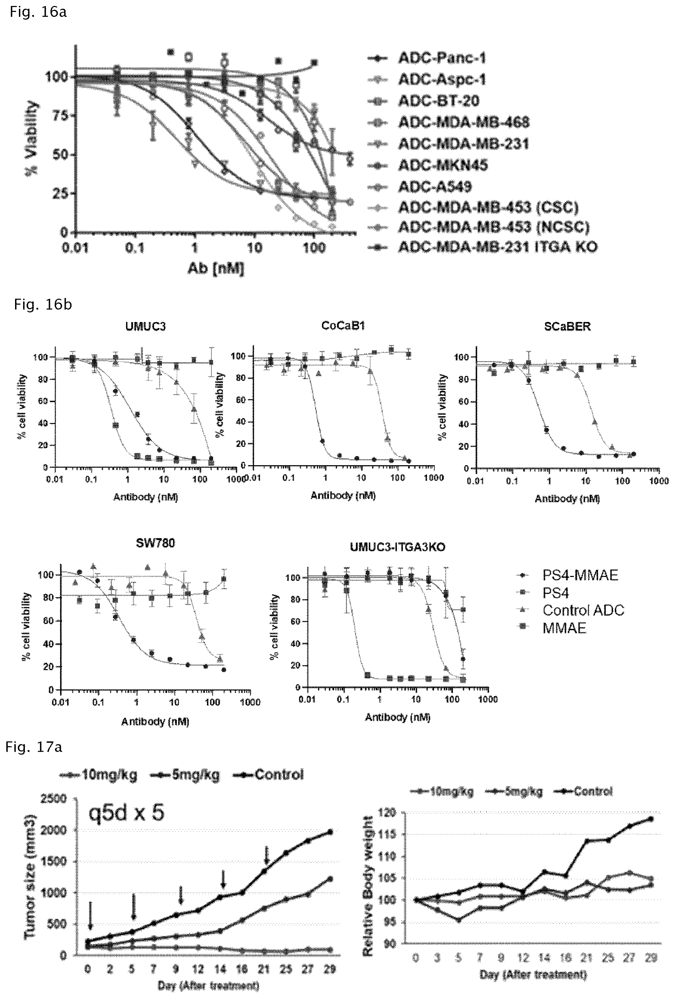

As a result, it was confirmed that PS4-vcMMAE, a PS4-drug conjugate according to one specific example, exhibits cell growth inhibition efficacy against various cancer cell lines (Fig. 16). Among them, the cell lines exhibiting the strongest cell growth inhibitory activity were confirmed to be breast cancer cell lines, especially triple-negative breast cancer MDA-MB-231 (IC50 $0.77 \pm 0.63$ nM), pancreatic cancer Panc-1 (IC50 $2.70 \pm 1.21$ nM), and lung cancer A549 (IC50 $5.22 \pm 2.17$ nM) cell lines. Additionally, the *in vitro* efficacy of the PS4 antibody-drug conjugate was confirmed in brain cancer and bladder cancer cell lines (Table 7). On the other hand, no anticancer efficacy was observed in breast cancer cell line (MDA-MB-231 ITGA KO) in which the ITGA3 subunit of the VLA-3 antigen targeted by the PS4 antibody was knocked out. This implies that the PS4-vcMMAE conjugate possesses efficacy in inhibiting the growth of target cancer cells. Furthermore, this implies that the PS4-drug conjugate according to one specific example possesses anticancer efficacy not only in breast cancer, including triple-negative breast cancer, and pancreatic cancer, but also in various cancer types such as lung cancer and gastric cancer, suggesting its potential application as an anticancer therapeutic agent.

[Table 7]

| Tissue | Cell line | Maximum (% inhibition) | $IC_{50}$ (nM) |
|---|---|---|---|
| TNBC | BT20 | $79.6 \pm 11.3$ | $133.6 \pm 43.38$ |
| TNBC | MDA-MB-468 | $89.88 \pm 2.5$ | - |

(continued)

| Tissue | Cell line | Maximum (% inhibition) | $IC_{50}$ (nM) |
|---|---|---|---|
| TNBC | MDA-MB-231 | 77.97±2.03 | 0.77±0.63 |
| TNBC | MDA-MB-231ITGA KO | n/a | n/a |
| TNBC | MDA-MB-453 | 96.2±0.36 | 9.79±1.39 |
| TNBC | MDA-MB-453 | 87.9±0.61 | 18.77±3.74 |
| Pancreatic | Panc-1 | 73.78±2.18 | 2.70±1.21 |
| Pancreatic | Aspc-1 | 51.78±25.44 | - |
| Stomach | MKN45 | 55.65±6.45 | 14.12±4.52 |
| Lung | A549 | 77.32±1.02 | 5.22±2.17 |
| bladder | UMUC-3 | 90.75±4.02 | 2.10±1.20 |
| bladder | CoCaB1 | 95.48±2.44 | 2.21±1.40 |
| bladder | SW780 | 90.43±2.32 | 1.78±1.05 |
| bladder | SCaBER | 89.71±0.61 | 1.88±0.79 |

**Example 12. Evaluation of the in vivo inhibitory efficacy of PS4 antibody-drug conjugate against triple-negative breast cancer.**

The *in vivo* efficacy evaluation of the PS4 antibody-drug conjugate according to one specific example was conducted as follows.

Triple-negative breast cancer (TNBC) MDA-MB-231 cells (2x10^6 cells) were mixed with Matrigel at a 1:1 (v/v) ratio and injected subcutaneously into BALB/c nude or NOD/SCID mice (Nara Bio). When the tumor volume reached 100 mm$^3$, the PS4-vcMMAE antibody-drug conjugate was administered intraperitoneally at 5 mg/kg or 10 mg/kg once every 5 days for a total of 5 times. 2-3 mice were used in each group. All animal experiments were conducted in accordance with protocols approved by the Institutional Animal Care and Use Committee (IACUC) of Kangwon National University (KW-210728-3).

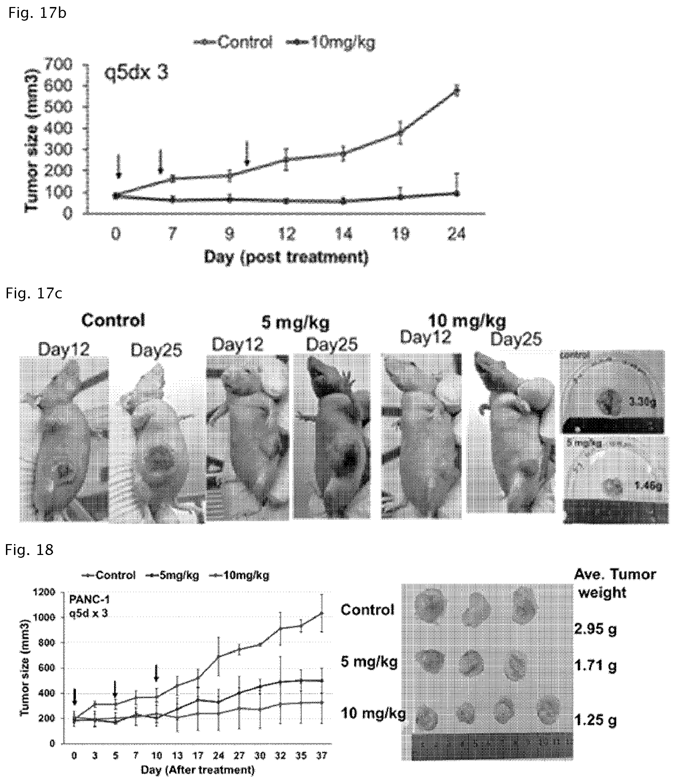

As a result, it was observed that tumor growth was inhibited in a PS4-MMAE dose-dependent manner in the experimental group administered the PS4-vcMMAE conjugate compared to the control group (Figs. 17a and 17c). In addition, it was confirmed that the size and weight of the extracted tumor tissue were reduced by approximately 55% in the experimental group treated with the PS4-vcMMAE conjugate. Furthermore, in the triple-negative breast cancer NOD/SCID experimental group administered the PS4 MDC drug at 10 mg/kg, it was confirmed that there was no significant difference in the tumor size compared to the time of drug administration (Fig. 17b). These results indicate that the PS4-vcMMAE conjugate according to one specific example has anticancer efficacy against triple-negative breast cancer.

**Example 13. Evaluation of the in vivo pancreatic cancer inhibitory efficacy of PS4 antibody-drug conjugates.**

The *in vivo* efficacy evaluation of the PS4 antibody-drug conjugate according to one specific example was conducted as follows.

PANC-1 ($3 \times 10^6$) cancer cells were subcutaneously injected into NOD/SCID (female 5-week-old) mice. When the tumor volume reached 100 m$^3$, the PS4-vcMMAE conjugate was intraperitoneally injected at 5 mg/kg or 10 mg/kg once every 5 days for a total of 3 times. The control group was treated with PBS, and the efficacy was evaluated with 3-4 mice per group. After the experiment, the tumors were excised, and the size of the tumor tissues in each experimental group was compared. All animal experiments were performed in accordance with the protocol approved by the Institutional Animal Care and Use Committee (IACUC) of Kangwon National University (KW-210728-3).

As a result, it was observed that tumor growth was inhibited in a PS4-MMAE dose-dependent manner in the experimental group administered the PS4-vcMMAE conjugate compared to the control group (Fig. 18). In addition, it was confirmed that the size and weight of the extracted tumor tissue were reduced in the experimental group treated with the PS4-vcMMAE conjugate. In particular, it was confirmed that the tumor size was reduced by at least 60% in the experimental group administered the 10 mg/kg PS4 MDC drug. Therefore, it was confirmed that the PS4 antibody-drug conjugate exhibits anticancer efficacy in triple-negative breast cancer and pancreatic cancer animal experimental models. These results indicate that the PS4-vcMMAE conjugate according to one specific example has anticancer efficacy in pancreatic cancer as well.

As demonstrated in the examples, it was verified that the PS4-drug conjugate according to one specific example exhibits the same efficacy in inhibiting tumor growth *in vivo* as *in vitro* efficacy verification, and it can also be predicted that the PS4-drug conjugate in which cytotoxic drugs other than MMAE is conjugated to the PS4 antibody will have anticancer efficacy in animal models of other species in which the antigen recognized by the PS4 antibody is overexpressed.

**Claims**

1. An antibody or a functional fragment thereof that is specific to a membrane protein of a cancer stem cell or a cancer cell, comprising:

    (a) a VH CDR1 comprising any one of the amino acid sequences of SEQ ID NOs: 1 to 4;
    (b) a VH CDR2 comprising any one of the amino acid sequences of SEQ ID NOs: 5 to 8;
    (c) a VH CDR3 comprising any one of the amino acid sequences of SEQ ID NOs: 9 to 12;
    (d) a VL CDR1 comprising any one of the amino acid sequences of SEQ ID NOs: 13 to 16;
    (e) a VL CDR2 comprising any one of the amino acid sequences of SEQ ID NOs: 17 to 20; and
    (f) a VL CDR3 comprising any one of the amino acid sequences of SEQ ID NOs: 21 to 24.

2. The antibody or functional fragment thereof according to claim 1, comprising:

    (a) a VH CDR1 of SEQ ID NO: 1, a VH CDR2 of SEQ ID NO: 5, a VH CDR3 of SEQ ID NO: 9, a VL CDR1 of SEQ ID NO: 13, a VL CDR2 of SEQ ID NO: 17, and a VL CDR3 of SEQ ID NO: 21;
    (b) a VH CDR1 of SEQ ID NO: 2, a VH CDR2 of SEQ ID NO: 6, a VH CDR3 of SEQ ID NO: 10, a VL CDR1 of SEQ ID NO: 14, a VL CDR2 of SEQ ID NO: 18, and a VL CDR3 of SEQ ID NO: 22;
    (c) a VH CDR1 of SEQ ID NO: 3, a VH CDR2 of SEQ ID NO: 7, a VH CDR3 of SEQ ID NO: 11, a VL CDR1 of SEQ ID NO: 15, a VL CDR2 of SEQ ID NO: 19, and a VL CDR3 of SEQ ID NO: 23; or
    (d) a VH CDR1 of SEQ ID NO: 4, a VH CDR2 of SEQ ID NO: 8, a VH CDR3 of SEQ ID NO: 12, a VL CDR1 of SEQ ID NO: 16, a VL CDR2 of SEQ ID NO: 20, and a VL CDR3 of SEQ ID NO: 24.

3. The antibody or functional fragment thereof according to claim 1, comprising:

    (a) a heavy chain variable region comprising an amino acid sequence selected from any one of SEQ ID NOs: 25 to 28, or a heavy chain variable region comprising a sequence having at least 90% homology to any one of SEQ ID NOs: 25 to 28; and
    (b) a light chain variable region comprising an amino acid sequence selected from any one of SEQ ID NOs: 29 to 32, or a light chain variable region comprising a sequence having at least 90% homology to any one of SEQ ID NOs: 29 to 32.

4. The antibody or functional fragment thereof according to claim 1, comprising:

    (a) a heavy chain variable region comprising the amino acid sequence of SEQ ID NO: 25 or a heavy chain variable region comprising a sequence at least 90% homologous to the amino acid sequence of SEQ ID NO: 25; and a light chain variable region comprising the amino acid sequence of SEQ ID NO: 29 or a light chain variable region comprising a sequence at least 90% homologous to the amino acid sequence of SEQ ID NO: 29;
    (b) a heavy chain variable region comprising the amino acid sequence of SEQ ID NO: 26 or a heavy chain variable region comprising a sequence at least 90% homologous to the amino acid sequence of SEQ ID NO: 26; and a light chain variable region comprising the amino acid sequence of SEQ ID NO: 30 or a light chain variable region comprising a sequence at least 90% homologous to the amino acid sequence of SEQ ID NO: 30;
    (c) a heavy chain variable region comprising the amino acid sequence of SEQ ID NO: 27 or a heavy chain variable region comprising a sequence at least 90% homologous to the amino acid sequence of SEQ ID NO: 27; An antibody or functional fragment thereof, comprising a light chain variable region comprising the amino acid sequence of SEQ ID NO: 31 or a light chain variable region comprising a sequence having at least 90% homology to the amino acid sequence of SEQ ID NO: 31; or
    (d) a heavy chain variable region comprising the amino acid sequence of SEQ ID NO: 28 or a heavy chain variable region comprising a sequence having at least 90% homology to the amino acid sequence of SEQ ID NO: 28; and a light chain variable region comprising the amino acid sequence of SEQ ID NO: 32 or a light chain variable region comprising a sequence having at least 90% homology to the amino acid sequence of SEQ ID NO: 32.

5. The antibody or functional fragment thereof according to claim 1, wherein the membrane protein of the cancer stem cell or cancer cell is ITGA3 (Integrin Subunit Alpha 3) or VLA3 (Integrin alpha 3 beta 1),

6. The antibody or functional fragment thereof according to claim 1, which is selected from the group consisting of VH, VL, IgG, Fab, Fab', F(ab')2, crossover Fab, scFab, dsFv, Fv, scFv, scFv-Fc, scFab-Fc, diabody, minibody, scAb, dAb, Fc-scFv, scFv-Fc-scFv, IgG-scFv, scFv-IgG, and combinations thereof.

7. The antibody or functional fragment thereof according to claim 6, which is a diabody, dAb, scFv or scFv-Fc.

8. The antibody or functional fragment thereof according to claim 6, wherein the scFv comprises a heavy chain variable region and a light chain variable region linked by a linker.

9. The antibody or functional fragment thereof according to claim 8, wherein the linker comprises the amino acid sequence of SEQ ID NO: 47.

10. The antibody or functional fragment thereof according to claim 6, wherein the Fc comprises an amino acid sequence derived from human, mouse, chicken, monkey, or camelid.

11. The antibody or functional fragment thereof according to claim 1, which is a chimeric antibody, a humanized antibody, or a fully human antibody.

12. The antibody or functional fragment thereof according to claim 1, which is a monoclonal antibody.

13. The antibody or functional fragment thereof according to claim 1, which is an IgG1, IgG2, IgG3, IgG4, or a combination thereof.

14. The antibody or functional fragment thereof according to claim 1, wherein a drug is conjugated to the antibody or functional fragment thereof.

15. The antibody or functional fragment thereof according to claim 14, wherein the drug is selected from cytotoxic agents, chemotherapeutic agents, anticancer agents, growth inhibitors, toxins, radioactive isotopes or combinations thereof.

16. A chimeric antigen receptor (CAR) protein comprising the antibody or functional fragment thereof according to claim 1.

17. An immune cell comprising the protein according to claim 16.

18. The immune cell according to claim 17, which is selected from the group consisting of T cells, NK (Natural Killer) cells, NKT (Natural Killer T) cells, monocytes, macrophages, and dendritic cells.

19. A pharmaceutical composition for treating or preventing cancer, comprising the antibody or functional fragment thereof according to claim 1, the protein according to claim 16, or the immune cell according to claim 17.

20. The pharmaceutical composition for treating or preventing cancer according to claim 19, wherein the cancer is selected from the group consisting of blood cancer, lung cancer, stomach cancer, liver cancer, bone cancer, pancreatic cancer, skin cancer, head and neck cancer, skin melanoma, uterine cancer, ovarian cancer, rectal cancer, brain cancer, colorectal cancer, colon cancer, breast cancer, triple negative breast cancer, uterine sarcoma, fallopian tube carcinoma, endometrial carcinoma, uterine cervical carcinoma, vaginal carcinoma, vulva carcinoma, esophageal cancer, laryngeal cancer, small intestine cancer, thyroid cancer, parathyroid cancer, soft-tissue sarcoma, urethral cancer, penile cancer, prostate cancer, chronic or acute leukemia, solid tumors in juvenile stage, differentiated lymphoma, bladder cancer, oral cavity carcinoma, renal cancer, renal cell carcinoma, renal pelvic carcinoma, primary central nervous system lymphoma, spinal axis tumors, glioma, brain stem glioma, and pituitary adenoma.

21. A polynucleotide encoding the antibody or functional fragment thereof according to claim 1.

22. A recombinant vector comprising the polynucleotide according to claim 21.

23. A recombinant cell comprising the vector according to claim 22.

24. A composition for diagnosing cancer, comprising the antibody or functional fragment thereof according to claim 1 or the polynucleotide according to claim 21.

25. A kit for diagnosing cancer comprising the antibody or functional fragment thereof according to claim 1 or the polynucleotide according to claim 21.

26. A reagent composition comprising the antibody or functional fragment thereof according to claim 1 or the polynucleotide according to claim 21.

Fig. 1

Fig. 2

Fig. 3

Fig. 4a

Fig. 4b

Fig. 4c

Fig. 4d

Fig. 4e

Fig. 5a

Fig. 5b

Fig. 6a

Fig. 6b

Fig. 6c
RU

Legend:
- 250 nM
- 125 nM
- 62.5 nM
- 31.3 nM
- 15.6 nM
- 7.8 nM
- 3.9 nM

X-axis: 100, 0, 100, 200, 300, 400, 500, 600, 700 Time s

Fig. 7a

Breast cancer: BT20, MDA-MB-453 (CSC), ZR-75-1, T47D (PS4-001 and Negative control Ab)

Normal cells: HEK293T, CHO-DG44, PBMC (PS4-001 and Negative control Ab)

Gastric Cancer: MKN45, NCI-87
Pancreatic Cancer: PANC-1, Aspc-1, Mia-paca-2
Colorectal Cancer: HCT-116
(PS4-001 and Negative control Ab)

Fig. 7b

Fig. 7c

Fig. 8

Expression of ITGA3 across TCGA cancers (with tumor and normal samples)

Fig. 9a

Expression of ITGA3 in BLCA based on Sample types

Fig. 9b

Expression of ITGA3 based on molecular subtypes of BLCA

Fig. 9c

Expression of ITGA3 in BLCA based on individual cancer stages

Fig. 10a

Expression of ITGA3 in PAAD based on Sample types

Fig. 10b

Expression of ITGA3 in PAAD based on individual cancer stages

Fig. 11a

Fig. 11b

## Pancreatic cancer

N=178, P<0.001

Fig. 11c

## Breast cancer (TNBC)

N=392, P=0.039

Fig. 12

● Cysteine residue

Fig. 13a

| Sample | Peak | Retention time(min) | Relative peak area(%) | DAR Distribution | Average DAR |
|---|---|---|---|---|---|
| PS4-002 | a | 24.918 | 100 | 0 | - |
| PS4-002-vcMMAE | A | 24.952 | 1.1 | 0 | |
| | B | 25.292 | 1.2 | 1 | |
| | C | 25.683 | 39.3 | 2 | 4.09 |
| | D | 27.032 | 10.4 | 4 | |
| | E | 28.180 | 48.0 | 6 | |

Fig. 13b

| Sample | Peak | Observed mass(Da) | ΔDelta mass(Da)* | Drug Distribution** |
|---|---|---|---|---|
| PS4-002 | a | 112,189.0 | - | 0 |
| PS4-002-vcMMAE | C | 115,066.0 | 2,877 | 2 |
| | D | 117,553.0 | 5,364 | 4 |
| | E | 119,921.0 | 7,732 | 6 |

*: Difference from observed mass of sample A.
**: Divided by the molecular mass of vcMMAE (1,316 Da).

Fig. 13c

| Peak | Retention time(min) | MW (kDa) | ratio | Identify | % Composition |
|---|---|---|---|---|---|
| A | 14.683 | 234.4 | 2.2 | HMW | 1.65 |
| B | 16.631 | 108.6 | 1.0 | monomer | 98.35 |

Fig. 13d

| Tissue | Cell line | Lot | Maximum (% inhibition) | IC$_{50}$ (nM) |
|---|---|---|---|---|
| TNBC | MDA-MB-231 | L043_003 | 80.4±1.87 | 0.57±0.39 |
| | | L043_016 | 80.9±0.72 | 0.61±0.37 |
| Pancreatic | Panc-1 | L043_003 | 78.5±1.52 | 1.92±0.54 |
| | | L043_016 | 78.1±1.24 | 1.1±0.19 |

Fig. 14a

Fig. 14b

Fig. 15

Fig. 16a

Fig. 16b

Fig. 17a

Fig. 17b

Fig. 17c

Fig. 18

## INTERNATIONAL SEARCH REPORT

| International application No. |
| --- |
| **PCT/KR2023/020864** |

| A. | CLASSIFICATION OF SUBJECT MATTER |
| --- | --- |

**C07K 16/28**(2006.01)i; **C07K 16/30**(2006.01)i; **A61K 39/00**(2006.01)i; **A61P 35/00**(2006.01)i; **G01N 33/574**(2006.01)i; **A61K 47/68**(2017.01)i

According to International Patent Classification (IPC) or to both national classification and IPC

| B. | FIELDS SEARCHED |
| --- | --- |

Minimum documentation searched (classification system followed by classification symbols)

C07K 16/28(2006.01); A61K 39/00(2006.01); C07K 14/725(2006.01); C07K 16/18(2006.01); C12N 15/13(2006.01); C12N 5/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인테그린(integrin), 항체(antibody), 막단백질(membrane protein), 암(cancer), 중쇄(heavy chain), 경쇄(light chain), 가변영역(variable region), 키메라 항원 수용체(chimeric antigen receptor), ITGA3, VLA3

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2018-520092 A (CYTOMX THERAPEUTICS, INC.) 26 July 2018 (2018-07-26)<br>See entire document. | 1-26 |
| A | JP 2018-065805 A (MILTENYI BIOTEC GMBH) 26 April 2018 (2018-04-26)<br>See entire document. | 1-26 |
| A | KR 10-2022-0150288 A (AETIO BIOTHERAPY, INC.) 10 November 2022 (2022-11-10)<br>See entire document. | 1-26 |
| A | NCBI. GenBank accession no. ACK76686.1 (24 July 2016).<br>See entire document. | 1-26 |
| A | NCBI. GenBank accession no. QFR45410.1 (25 February 2020).<br>See entire document. | 1-26 |

☑ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **22 March 2024** | **25 March 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/020864**

| C. | DOCUMENTS CONSIDERED TO BE RELEVANT | |
|---|---|---|
| Category* | Citation of document, with indication, where appropriate, of the relevant passages | Relevant to claim No. |
| PX | KR 10-2574148 B1 (NIOBIOPHARMACEUTICALS. INC.) 05 September 2023 (2023-09-05) See claims 1-24. ※ This document is the published patent of a priority application of the present international application. | 1-26 |

Form PCT/ISA/210 (second sheet) (July 2022)

**INTERNATIONAL SEARCH REPORT**

| International application No. |
| --- |
| **PCT/KR2023/020864** |

| Box No. I    Nucleotide and/or amino acid sequence(s) (Continuation of item 1.c of the first sheet) |
| --- |

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

EP 4 703 380 A1

| INTERNATIONAL SEARCH REPORT<br>Information on patent family members | International application No.<br><br>**PCT/KR2023/020864** |

| Patent document<br>cited in search report | | | Publication date<br>(day/month/year) | Patent family member(s) | | | Publication date<br>(day/month/year) |
|---|---|---|---|---|---|---|---|
| JP | 2018-520092 | A | 26 July 2018 | AU | 2016-258988 | A1 | 07 December 2017 |
| | | | | CA | 2984892 | A1 | 10 November 2016 |
| | | | | CN | 107922490 | A | 17 April 2018 |
| | | | | EP | 3292151 | A1 | 14 March 2018 |
| | | | | US | 10233244 | B2 | 19 March 2019 |
| | | | | US | 2016-0355592 | A1 | 08 December 2016 |
| | | | | US | 2019-0309072 | A1 | 10 October 2019 |
| | | | | WO | 2016-179335 | A1 | 10 November 2016 |
| JP | 2018-065805 | A | 26 April 2018 | CA | 2982963 | A1 | 20 April 2018 |
| | | | | CN | 107964046 | A | 27 April 2018 |
| | | | | EP | 3311832 | A1 | 25 April 2018 |
| | | | | EP | 3735983 | A1 | 11 November 2020 |
| | | | | EP | 3738607 | A1 | 18 November 2020 |
| | | | | JP | 7222600 | B2 | 15 February 2023 |
| | | | | US | 10617720 | B2 | 14 April 2020 |
| | | | | US | 2019-0209611 | A1 | 11 July 2019 |
| | | | | US | 2020-0282035 | A1 | 10 September 2020 |
| | | | | US | 2021-0085769 | A1 | 25 March 2021 |
| KR | 10-2022-0150288 | A | 10 November 2022 | AU | 2021-208642 | A1 | 28 July 2022 |
| | | | | CA | 3165414 | A1 | 22 July 2021 |
| | | | | CN | 114945597 | A | 26 August 2022 |
| | | | | EP | 4090686 | A2 | 23 November 2022 |
| | | | | JP | 2023-510601 | A | 14 March 2023 |
| | | | | US | 2021-0301015 | A1 | 30 September 2021 |
| | | | | WO | 2021-146590 | A2 | 22 July 2021 |
| | | | | WO | 2021-146590 | A3 | 11 November 2021 |
| KR | 10-2574148 | B1 | 05 September 2023 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5260203 A
- US 5585089 A, Queen
- US 5892019 A

**Non-patent literature cited in the description**

- **HAMERS-CASTERMAN et al.** *Nature*, 1993, vol. 363, 446-448
- **KABAT, E. et al.** Sequences of Proteins of Immunological Interest. U.S. Department of Health and Human Services, 1983
- **CHOTHIA** ; **LESK**. *J. Mol. Biol.*, 1987, vol. 196, 901-917
- **KABAT et al.** Sequences of Proteins of Immunological Interest. Public Health Service, National Institutes of Health, 1991
- **LEFRANC et al.** *Nucl. Acids Res.*, 1999, vol. 27, 209-212
- **RUIZ et al.** *Nucl. Acids Res.*, 2000, vol. 28, 219-221
- **HONNEGER** ; **PLUNKTHUN**. *Mol. Biol.*, 2001, vol. 309, 657-670
- **KABAT et al.** Sequences of Proteins of Immunological Interest. U.S. Dept. of Health and Human Services, 1983
- **ROUX et al.** *J. Immunol*, 1998, vol. 161, 4083
- **KABAT, E.A. et al.** Sequences of Proteins of Immunological Interest. U.S. Dept. of Health and Human Services, 1991
- Immunology-A Synthesis. Sinauer Associates, 1991
- **RUSSELL et al.** *J. Mol Biol.*, 1994, vol. 244, 332-350
- **SAMBROOK et al.** Molecular Cloning: A Laboratory Manual. Cold Spring Harbor Laboratory Press, 2012
- **AUSUBEL et al.** Current Protocols in Molecular Biology. Greene Publishing Associates
- **KIM, E.G et al.** *Conjugates. Biomolecules*, 2020, vol. 10, 955
- **KIM, E.G. et al.** *Biomolecules*, 2020, vol. 10, 955
- **PETERSON RJ** ; **KOVAL M**. Above the Matrix: Functional Roles for Apically Localized Integrins.. *Front. Cell Dev. Biol.*, 2021, vol. 9, 699407
- *Mol Cancer Ther*, 2008, vol. 7 (8), 2486-2497